# EUROPEAN PATENT APPLICATION

(11) **EP 4 406 977 A1**
(43) Date of publication of application: **31.07.2024**
(21) Application number: 22872546.1
(22) Date of filing: 26.07.2022
(51) Int. Cl.: C08B 37/16, A61K 45/00, A61K 47/69, C08B 30/00, C08B 30/18

(54) **PRODUCTION METHOD OF CYCLIC OLIGOSACCHARIDE, CYCLIC OLIGOSACCHARIDE AND COMPLEXATION AGENT**

(30) Priority: 22.09.2021 JP 2021154167
(71) Applicant: National University Corporation Tottori University, Tottori-shi Tottori 680-8550 (JP); Koganei Corporation, Koganei-shi Tokyo 184-8533 (JP)
(72) Inventor: NOKAMI Toshiki, Tottori-shi, Tottori 680-8550 (JP); HAMADA Tomoaki, Koganei-shi, Tokyo 184-8533 (JP); MAJIMA Kazuhiro, Koganei-shi, Tokyo 184-8533 (JP); KAWANO Takahiro, Koganei-shi, Tokyo 184-8533 (JP); MURAMOTO Yasuhiko, Koganei-shi, Tokyo 184-8533 (JP)
(74) Representative: Zimmermann & Partner Patentanwälte mbB
(86) International application number: PCT/JP2022/028680
(87) International publication number: WO 2023/047790

(57) **Abstract**

The object is to provide a method for producing a cyclic oligosaccharide through which a cyclic oligosaccharide can be efficiently synthesized. In addition, the object is to provide a novel cyclic oligosaccharide obtained through the production method. The method for producing a cyclic oligosaccharide includes subjecting a linear oligosaccharide, in which 5 to 10 α-glucosamines or derivatives thereof are bonded in series via 1,4-bonds, to a liquid phase electrolysis reaction.

## Description

### [Technical Field]

The present invention relates to a method for producing a cyclic oligosaccharide and a novel cyclic oligosaccharide. In addition, the present invention relates to an inclusion agent using the oligosaccharide.

### [Background Art]

A cyclic oligosaccharide refers to molecules in which an oligosaccharide composed of a plurality of monosaccharides is cyclized. It is known that these molecules have hydrophobic cavities within the molecules and can encapsulate organic molecules and ions in the cavities. A typical cyclic oligosaccharide is cyclodextrin (NPL 1) (Formula 11). In addition, there are also cyclic oligosaccharides such as cycloawaodorin (NPL 2) synthesized by a chemical synthesis method (Formula 12). Since these molecules have an inclusion ability, these are expected to be applied to foods, pharmaceuticals, drug delivery systems and the like. In addition, depending on the size of the cavity, there may be a difference in the molecules that can be encapsulated. Therefore, the control of the cavities of the cyclic oligosaccharides, that is, the design and synthesis of oligosaccharides, is important.

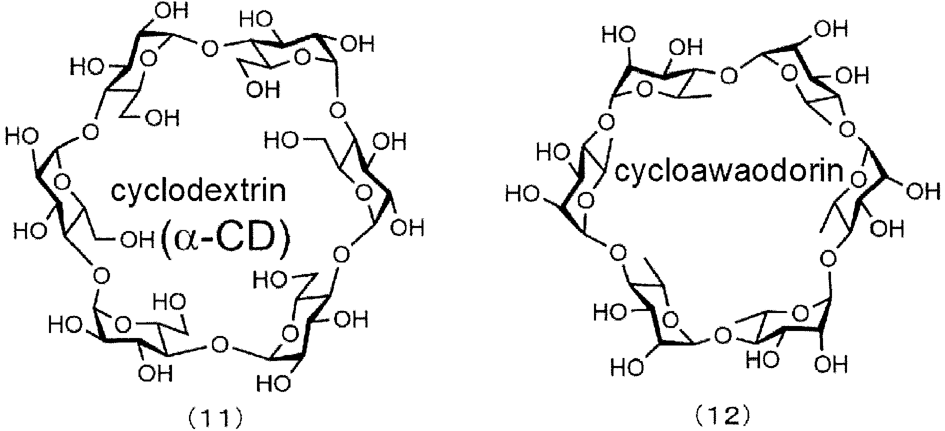

### [Typical cyclic oligosaccharides]

### [Citation List]

### [Non Patent Literature]

[NPL 1] Ogawa, T.; Takahashi, Y. Carbohydr. Res 1985, 138, C5.
[NPL 2] Nishizawa, M.; Imagawa, H.; Kan, Y.; Yamada, H.; Tetrahedron Lett. 1991, 32, 5551.

### [Summary of Invention]

### [Technical Problem]

In recent years, the synthesis of small cyclodextrins, the sterically distorted cyclodextrins of CD3 and CD4, has been achieved (Ikuta, D.; Hirata, Y.; Wakamori, S.; Shimada, H. Tomabechi, Y.; Kawasaki, Y.; Ikeuchi, K.; Hagimori, T.; Matsumoto, S.; Yamada, H. Science 2019, 364, 674.) (Formulae 13 and 14). For the development and application of the functions of these cyclic oligosaccharides, enzyme synthesis methods alone do not suffice, hence the development of chemical methods through which supply can be performed stably is strongly desired.

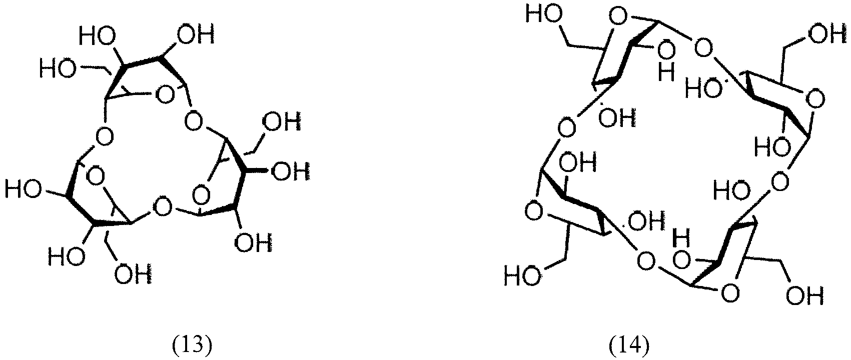

Therefore, an object of the present invention is to provide a method for producing a cyclic oligosaccharide through which a cyclic oligosaccharide can be efficiently synthesized. In addition, an object of the present invention is to provide a novel cyclic oligosaccharide obtained through the production method.

### [Solution to Problem]

In view of the above circumstances, the inventors conducted studies, and as a result, have found that a desired cyclic oligosaccharide can be synthesized by performing a liquid phase electrolytic method using linear oligosaccharides bonded in series via 1,4-bonds.

Specifically, the above problem was addressed by the following aspects.
[1] A method for producing a cyclic oligosaccharide, the method including
   subjecting a linear oligosaccharide, in which 5 to 10 α-glucosamines or derivatives thereof are bonded in series via 1,4-bonds, to a liquid phase electrolysis reaction.
[2] The method for producing a cyclic oligosaccharide according to [1],
   wherein the linear oligosaccharide is represented by the following Formula (1): 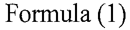 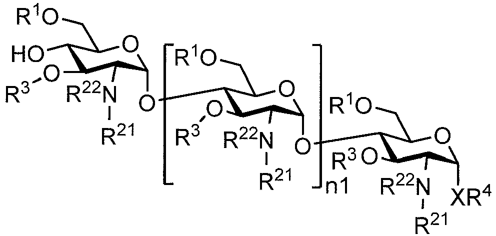 (in Formula (1), R¹'s are each independently a protecting group with a formula weight of 500 or less, R²¹ and R²² are each independently a hydrogen atom or a protecting group with a formula weight of 300 or less, R³'s are each independently a protecting group with a formula weight of 300 or less, R⁴ is a substituent, X is a sulfur atom, a selenium atom, or a tellurium atom, and n1 is an integer of 3 to 8; and R²² and R³ that are bonded to the same ring may be bonded to each other to form a ring).
[3] The method for producing a cyclic oligosaccharide according to [1],
   wherein the linear oligosaccharide is represented by Formula (1-1) below: 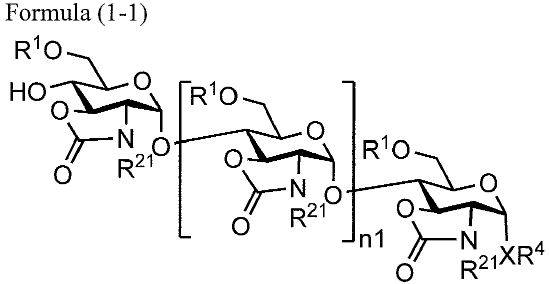 (in Formula (1-1), R¹'s are each independently a protecting group with a formula weight of 500 or less, R²¹'s are each independently a hydrogen atom or a protecting group with a formula weight of 300 or less, R⁴ is a substituent, X is a sulfur atom, a selenium atom, or a tellurium atom, and n1 is an integer of 3 to 8).
[4] A cyclic oligosaccharide represented by Formula (2): 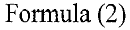 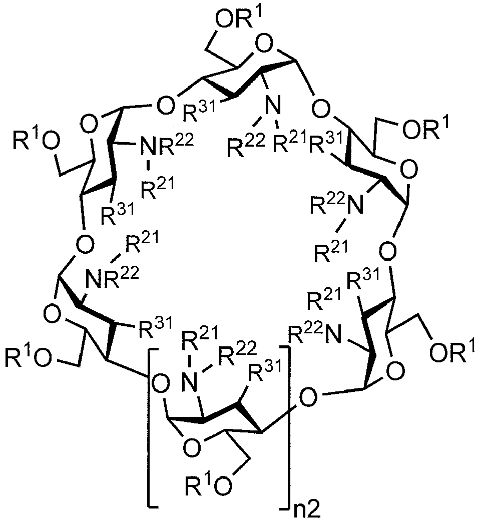 (in Formula (2), R¹'s are each independently a protecting group with a formula weight of 500 or less, R²¹ and R²² are each independently a hydrogen atom or a protecting group with a formula weight of 300 or less, R³¹ is an R³-O- group, R³'s are each independently a protecting group with a formula weight of 300 or less, and n2 is an integer of 0 to 5; and R²² and R³¹ that are bonded to the same ring may be bonded to each other to form a ring).
[5] A cyclic oligosaccharide represented by Formula (2-1): 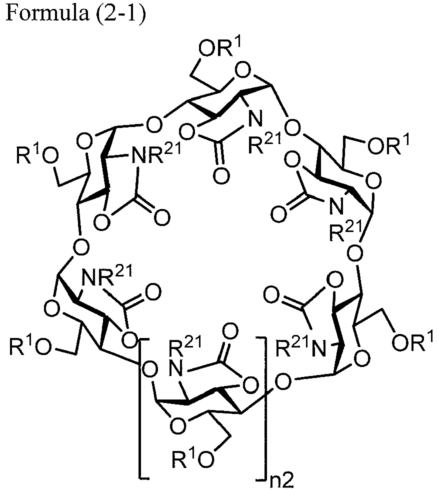 (in Formula (2-1), R¹'s are each independently a protecting group with a formula weight of 500 or less, R²¹'s are each independently a hydrogen atom or a protecting group with a formula weight of 300 or less, and n2 is an integer of 0 to 5).
[6] A cyclic oligosaccharide represented by Formula (3): 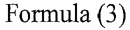 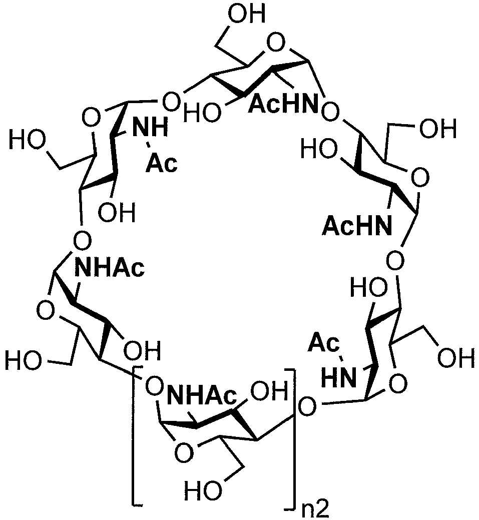 (in Formula (3), n2 represents an integer of 0 to 5, and Ac represents an acetyl group).
[7] A cyclic oligosaccharide represented by Formula (4): 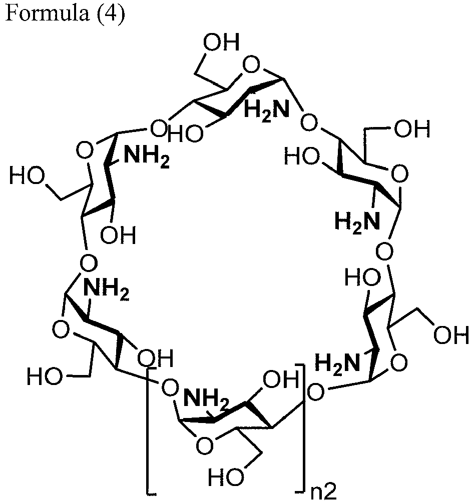 (in Formula (4), n2 represents an integer of 0 to 5).
[8] An inclusion agent containing the compound according to [7].
[9] A method for producing a cyclic oligosaccharide, the method including
   opening an oxazolidinone ring of a cyclic oligosaccharide represented by Formula (2-1) below with a base, reacting an obtained sugar with acetic anhydride, acetylating the ring-opened site, additionally reacting with K₂CO₃ to obtain a sugar in which an acetyloxy group at the 3-position is converted into a hydroxyl group, reacting the sugar with an acid to obtain a sugar of Formula (3) in which a group for R¹ is a hydrogen atom and the substituent at the 6-position is a hydroxyl group: 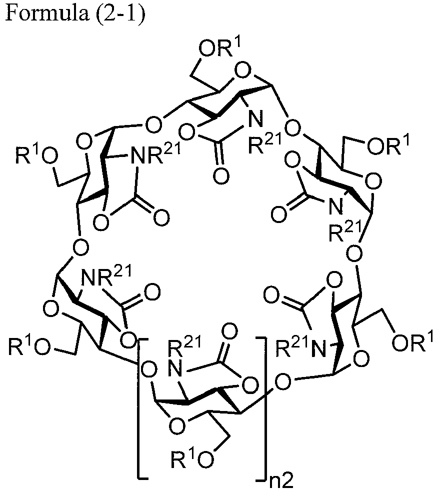 (in Formula (2-1), R¹'s are each independently a protecting group with a formula weight of 500 or less, R²¹'s are each independently a hydrogen atom or a protecting group with a formula weight of 300 or less, and n2 is an integer of 0 to 5) 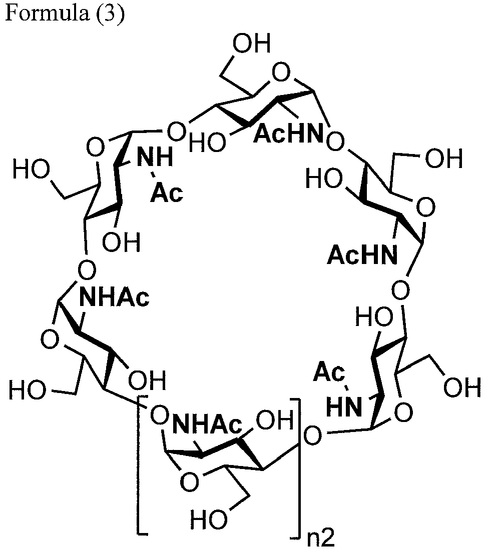 (in Formula (3), n2 represents an integer of 0 to 5, and Ac represents an acetyl group).

### [Advantageous Effects of Invention]

According to the production method of the present invention, it is possible to efficiently synthesize cyclic oligosaccharides. In addition, it is possible to provide novel cyclic oligosaccharides obtained through the production method.

### [Brief Description of Drawings]

[Fig. 1]

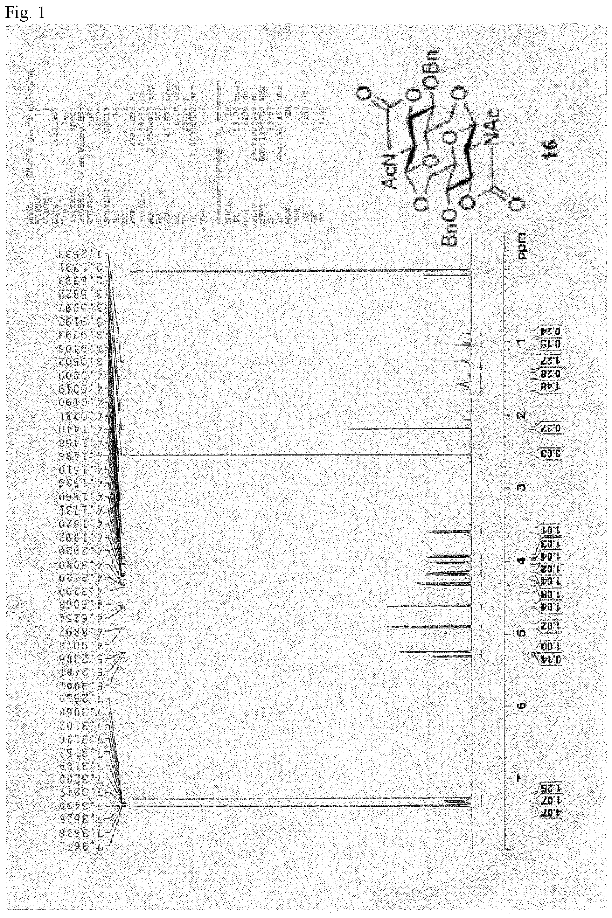

   Fig. 1 shows an NMR spectrum of compound 16 (part 1).
[Fig. 2]

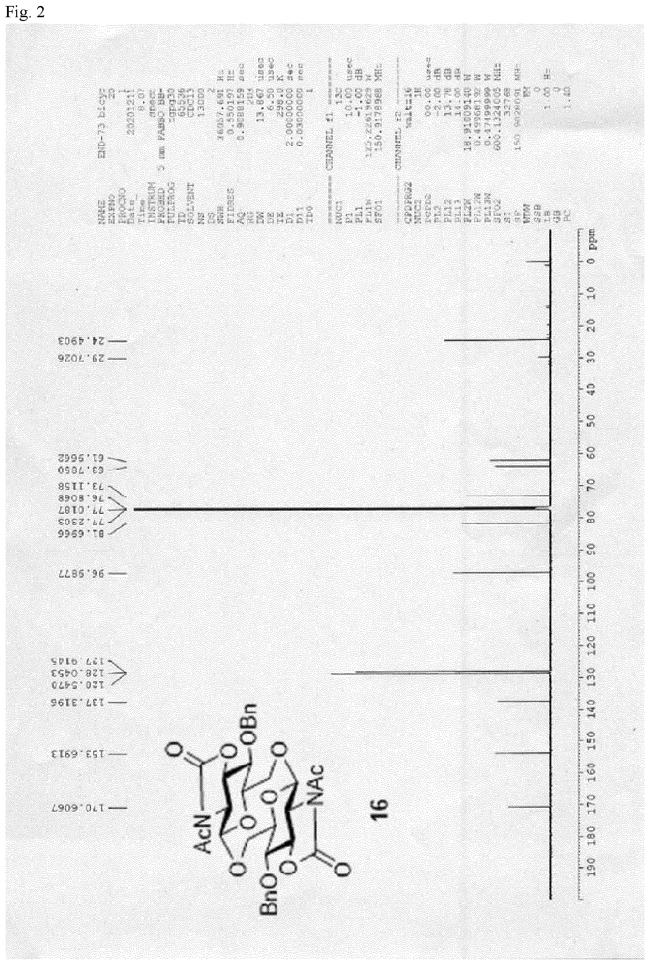

   Fig. 2 shows an NMR spectrum of compound 16 (part 2).
[Fig. 3]

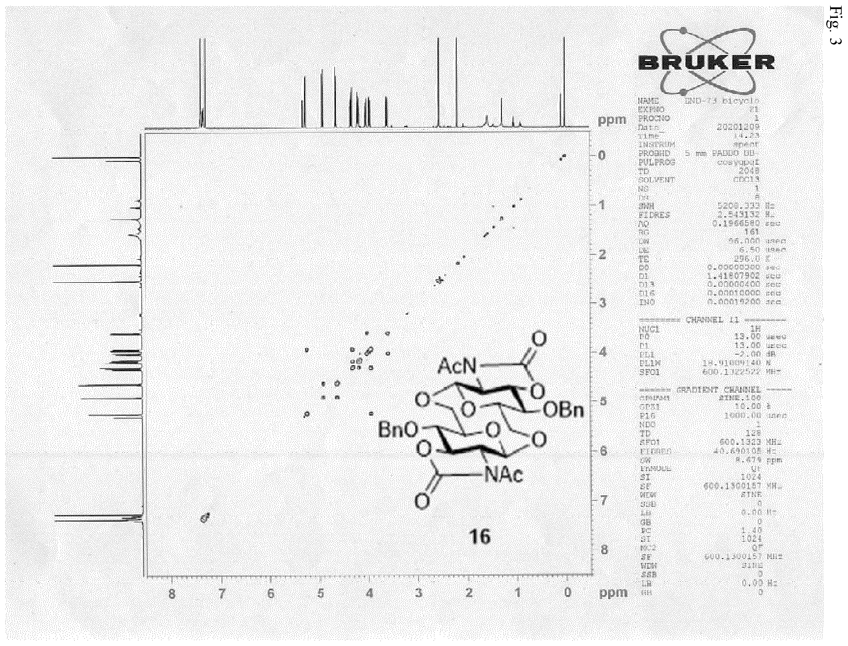

   Fig. 3 shows an NMR spectrum of compound 16 and its analysis diagram (part 3).
[Fig. 4]

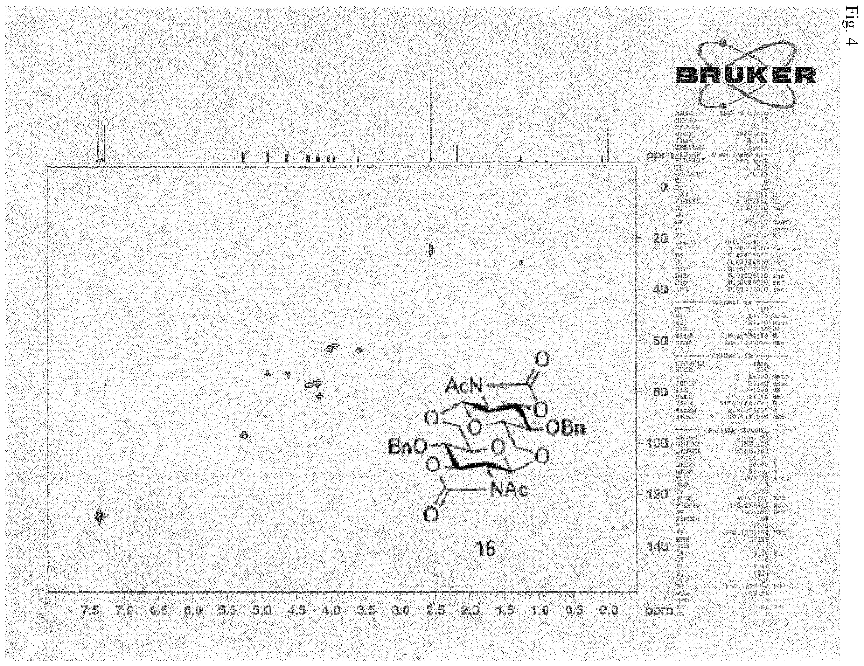

   Fig. 4 shows an NMR spectrum of compound 16 and its analysis diagram (part 4).
[Fig. 5]

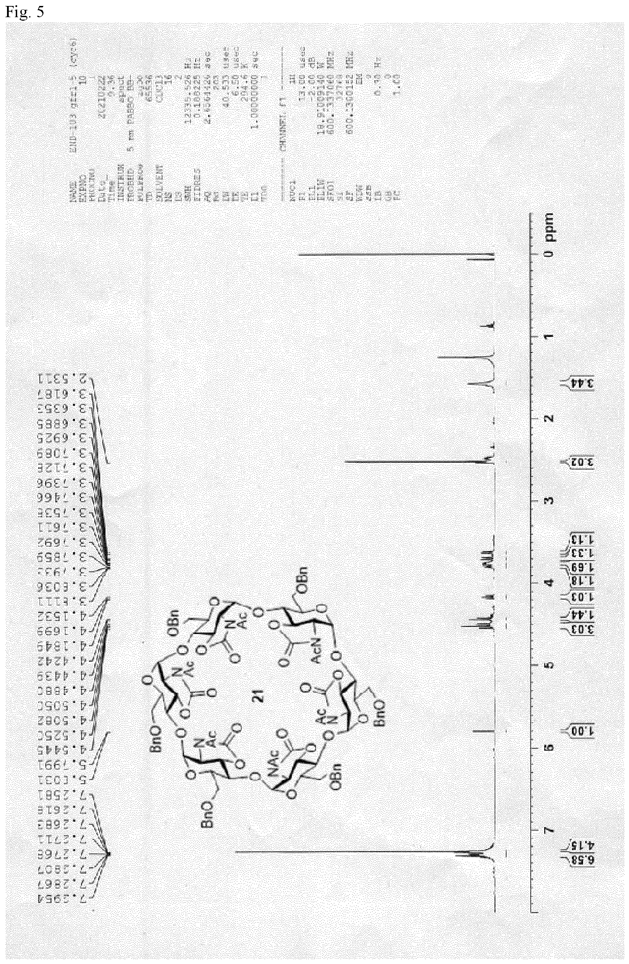

   Fig. 5 shows an NMR spectrum of compound 21 (part 1).
[Fig. 6]

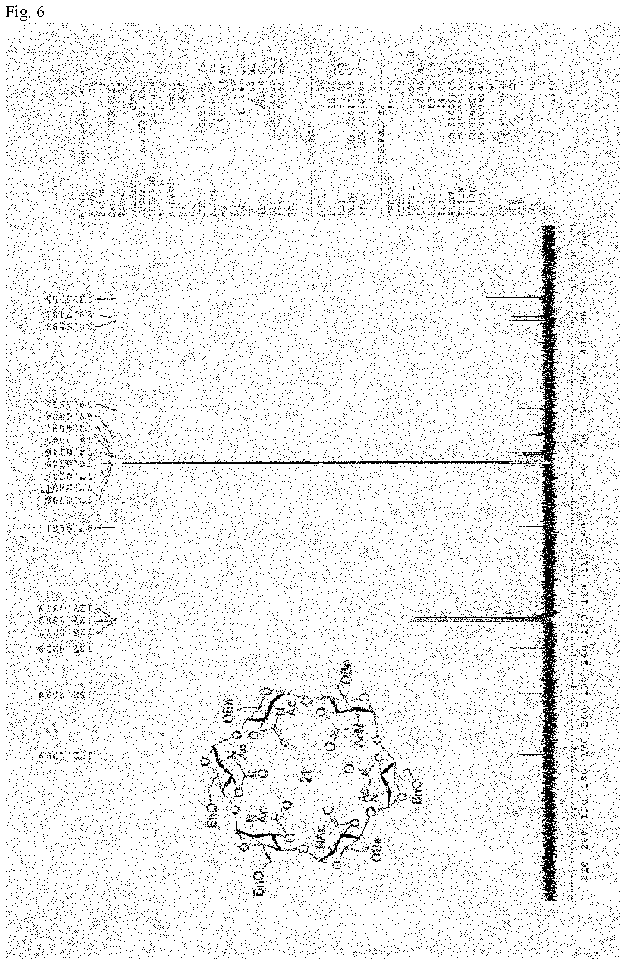

   Fig. 6 shows an NMR spectrum of compound 21 (part 2).
[Fig. 7]

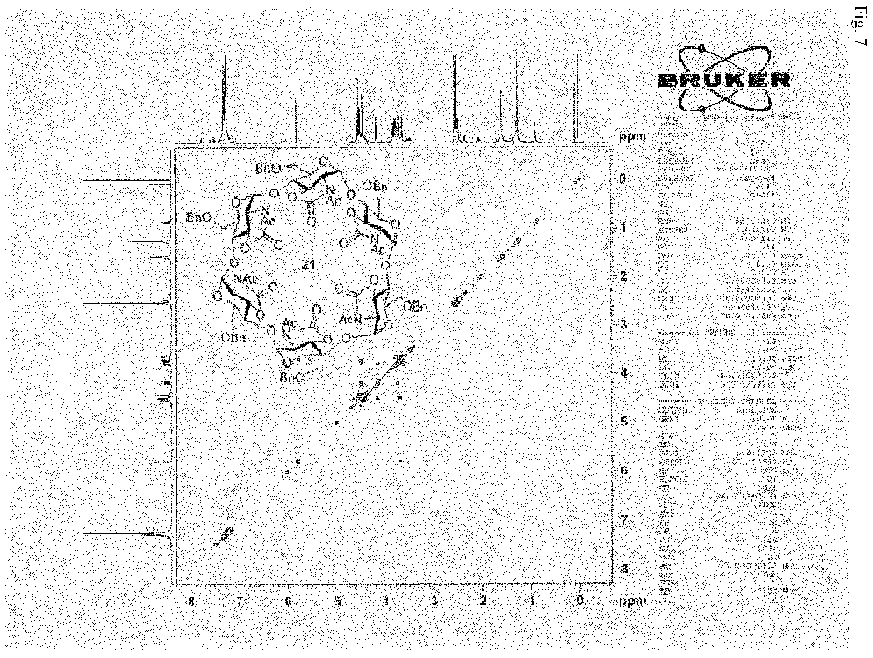

   Fig. 7 shows an NMR spectrum of compound 21 and its analysis diagram (part 3).
[Fig. 8]

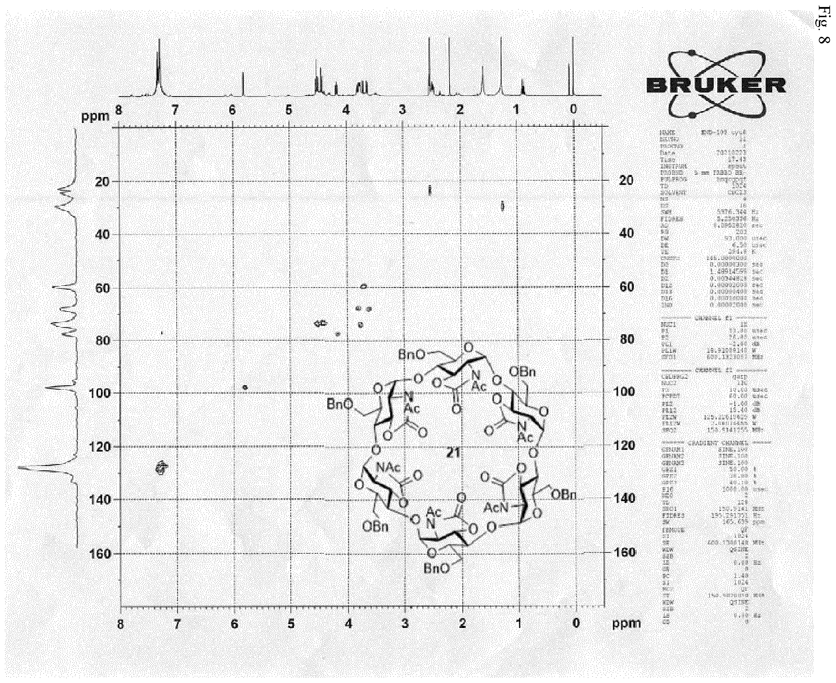

   Fig. 8 shows an NMR spectrum of compound 21 and its analysis diagram (part 4).
[Fig. 9]

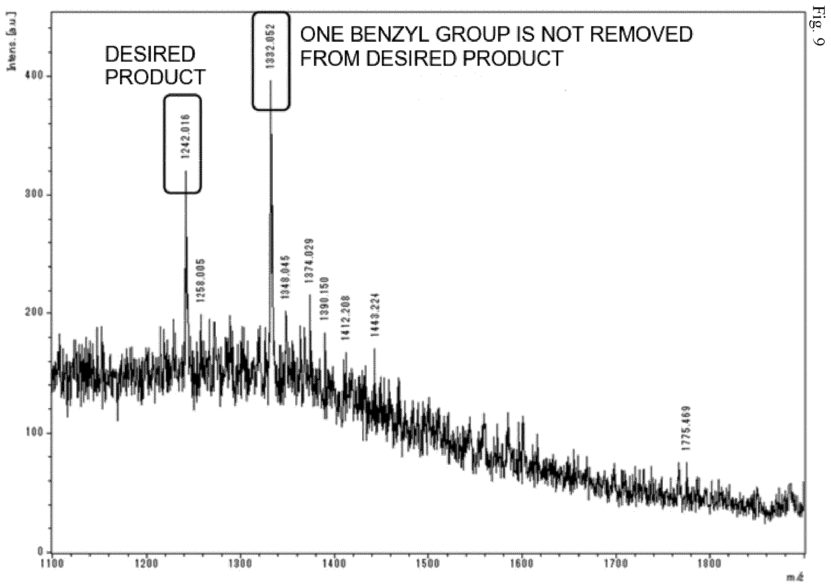

   Fig. 9 shows MALDI-TOF MS after reaction termination in a reaction scheme (27).
[Fig. 10]

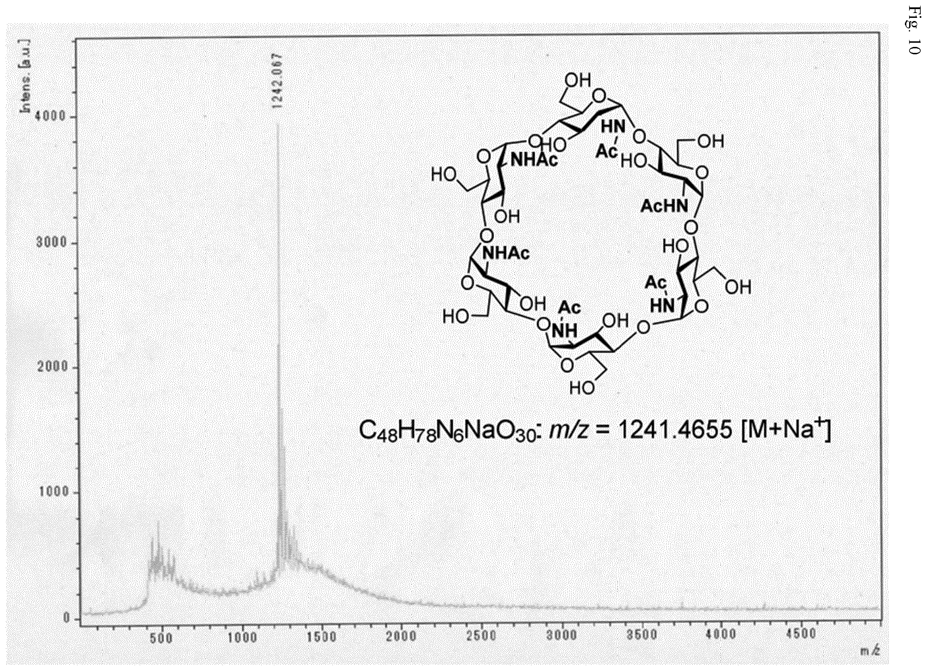

   Fig. 10 shows MALDI-TOF MS after gel filtration of a reaction product (saccharide 26) in the reaction scheme (27).
[Fig. 11]

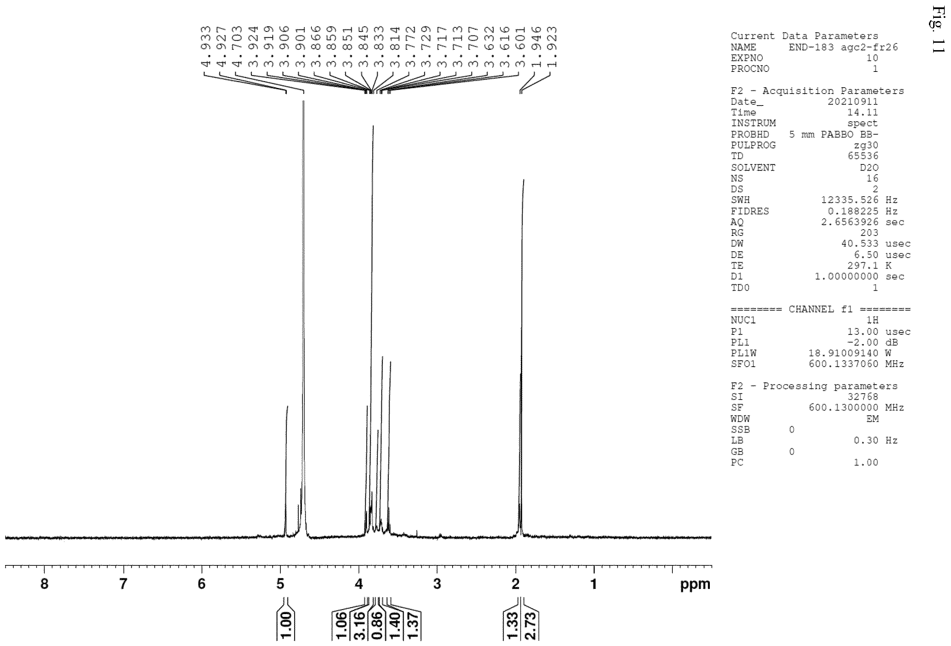

   Fig. 11 shows ¹H-NMR after gel filtration of the reaction product (saccharide 26) in the reaction scheme (27).
[Fig. 12]

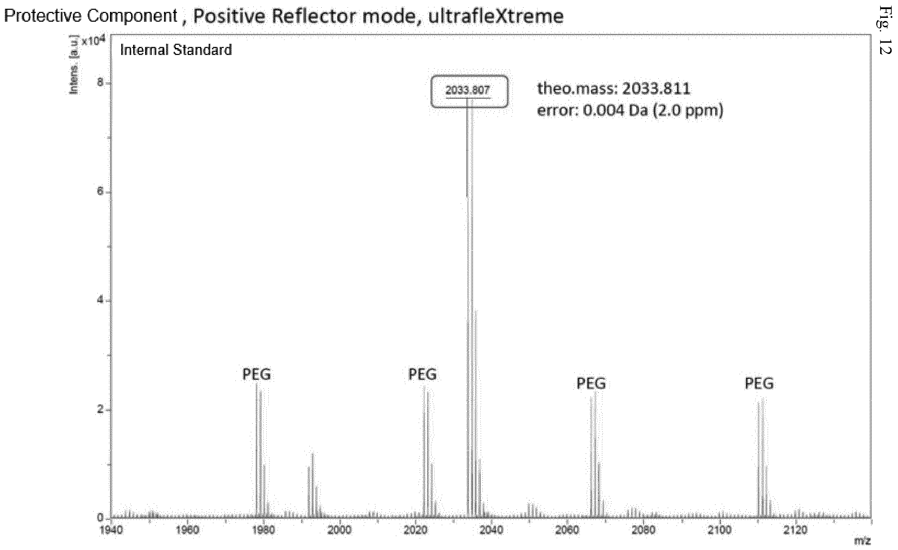

   Fig. 12 is a graph showing the results of mass spectrometry measurement of saccharide 24, which is an intermediate.
[Fig. 13]

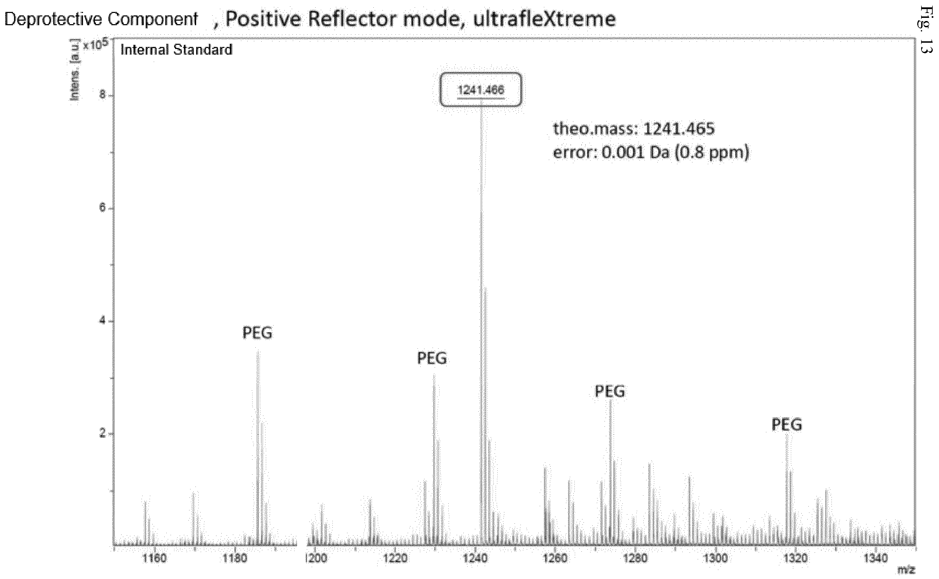

   Fig. 13 is a graph showing the results of mass spectrometry measurement of saccharide 26, which is the final product.

### [Description of Embodiments]

Hereinafter, a form for implementing the present invention (hereinafter simply referred to as "the present embodiment") will be described in detail. Here, the present embodiment below is an example for describing the present invention, and the present invention is not limited only to the present embodiment.

Here, in this specification, "to" is used to indicate numerical values stated before and after "to" as a lower limit value and an upper limit value.

In this specification, various physical property values and characteristic values are values at 23°C unless otherwise specified.

In the expression of groups (atom groups) in this specification, expressions that are not indicated as substituted or unsubstituted include groups having no substituents (atom groups) as well as groups having a substituent (atom groups). For example, the term "alkyl group" includes not only an alkyl group having no substituents (unsubstituted alkyl groups) but also an alkyl group having a substituent (substituted alkyl group). In this specification, for expressions that are not indicated as substituted or unsubstituted, unsubstituted is preferable.

When standards shown in this specification differ in measurement methods and the like depending on the year, unless otherwise specified, standards as of January 1, 2021, are used.

Regarding abbreviations for substituents and the like, Et is an ethyl group, Bn is a benzyl group, Ar is an aryl group, Phth is a phthaloyl group, MeOH is methanol, Ph is a phenyl group, Ac is an acetyl group, DMPA is 4-dimethylaminopyridine, Tf is a trifluoromethanesulfonyl group, TfOH is trifluoromethanesulfonic acid, TMSOTf is trimethylsilyltrifluoromethanesulfonate, THF is tetrahydrofuran, Bu₄NOTf is tetrabutylammonium triflate, TBDPS is a tert-butyldiphenylsilyl group, and DTBMP is 2,6-di-tert-butyl-4-methylpyridine.

First, a synthetic pathway for monosaccharide building blocks, which have long been studied as monomer candidates for polymerization reactions, will be described (reaction scheme 1). Using glucosamine hydrochloride 1 as a starting material, a phthaloyl protection of an amino group was performed with phthalic anhydride, and subsequently, acetylation with Ac₂O was performed. Next, according to the reaction with 4-chlorothiophenol, conversion into thioglycoside 4 was performed. This thioglycoside was deacetylated under acidic conditions, and benzylidene protection was then performed with benzaldehyde dimethyl acetal to synthesize a compound 6. Then, the hydroxy group at the 3-position was acetylated again with acetic anhydride to obtain a compound 7. Then, the compound 7 was reacted with trimethylsilyl triflate in a borane THF complex, benzylidene acetal was cleaved, and a monosaccharide building block 8 was synthesized.

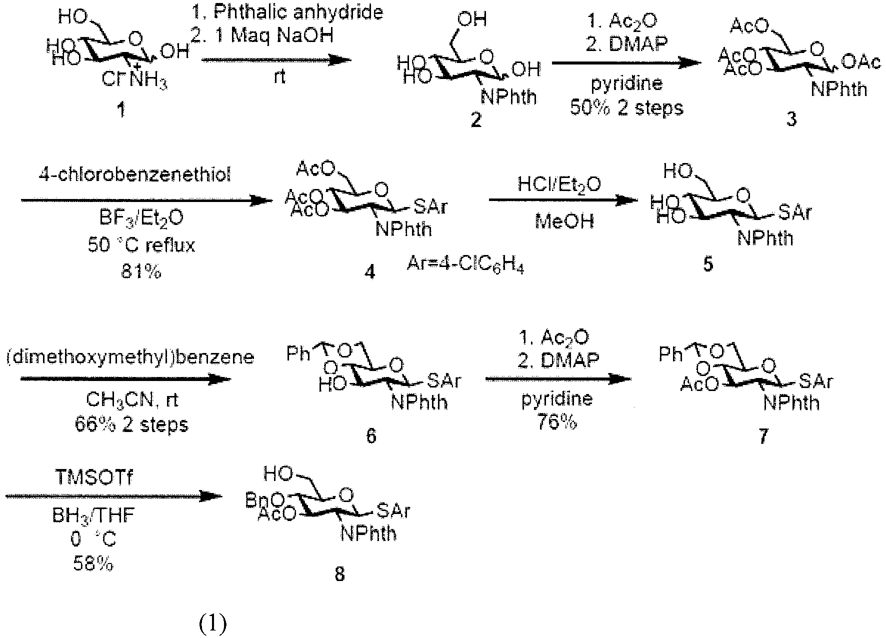

### [Synthesis of monosaccharide building blocks studied so far (refer to the following literature)]

·Manmode, S.; Tanabe, S.; Yamamoto, T.; Sasaki, N.; Nokami, T.; Itoh, T. Chemistry Open 2019, 8, 869.
·Yano, K.; Itoh, T.; Nokami. Carbohydr. Res. 2020, 492, 108018.

Using the obtained monosaccharide building block 8, synthesis of cyclic saccharides by an electrolytic polymerization method was attempted (reaction scheme 2). The reaction conditions were as follows. After electrolytic oxidation was performed under a constant current condition at -60C°, the temperature was raised to -40°C, and a glycosylation reaction was performed for 3,600 seconds. Then, Et₃ was added to terminate the reaction.

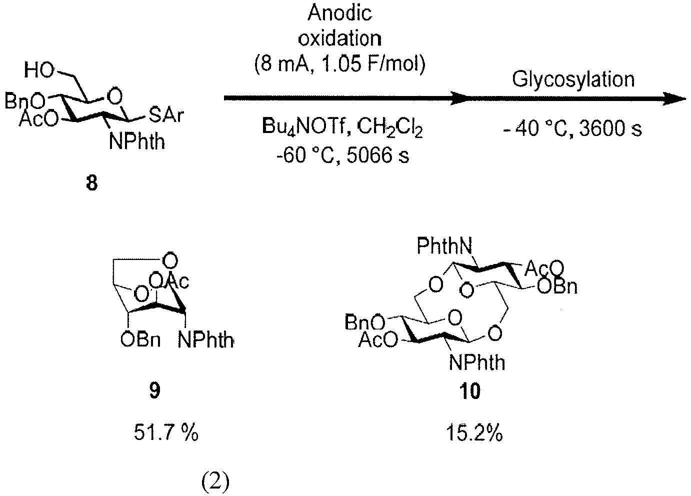

### Anodic oxidation

### Glycosylation

When the reaction was performed under the reaction conditions, cyclic tri- or higher saccharides could not be obtained, but 1,6-anhydro saccharide 9 and cyclic disaccharide 10 were obtained. The following mechanism is proposed as the reaction mechanism by which this 1,6-anhydro saccharide is obtained (reaction scheme 3). It is thought that the α-triflate intermediate generated during electrolytic oxidation undergoes a conformational change and undergoes intramolecular glycosylation before coupling with other intermediates. Therefore, it is thought that it is important to prevent a conformation that would produce 1,6-anhydro saccharide in order to obtain larger cyclic saccharides.

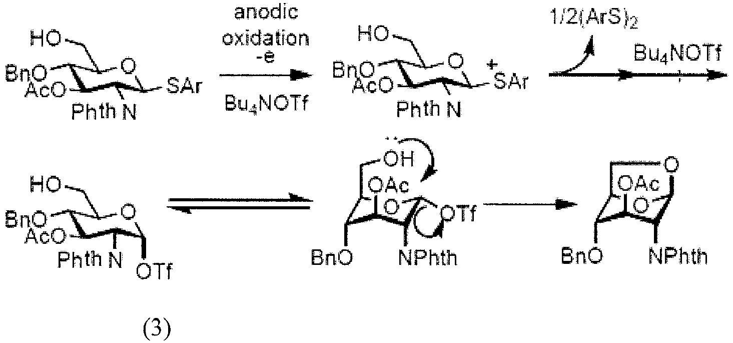

### [Reaction mechanism of side reaction that may occur during electrolytic polymerization]

### Polymerization reaction using substrate with oxazolidinone protecting groups introduced into the 2- and 3-positions and its problems

In order to inhibit side reactions due to stereoinversion of a sugar pyranose ring, it was attempted to introduce protecting groups that become bulky upon inversion, that is, 2,3-oxazolidinone protection, at 2- and 3-positions, where introduction is easiest (reaction scheme 4). Using the monosaccharide building block 8 used above as a starting material, synthesis was attempted. The 6-position of the monosaccharide building block 8 was protected with a TBDPS group, and conversion into a compound 11 was performed. Then, in order to deprotect the phthalimide protecting group, deprotection was performed using dehydrated ethylenediamine to obtain a compound 12. In this case, the acetyl group at the 3-position was also deprotected at the same time. Next, oxazolidinone protection at the 2- and 3-positions was performed in CH₂Cl₂ and a 10% NaHCO₃ aqueous solution, and conversion into a compound 13 was performed. Then, acetyl protection on nitrogen was performed in DMF, and a TBDPS protecting group at the 6-position was then deprotected to obtain an oxazolidinone protective component 15 in which the hydroxyl group at the 6-position was unprotected.

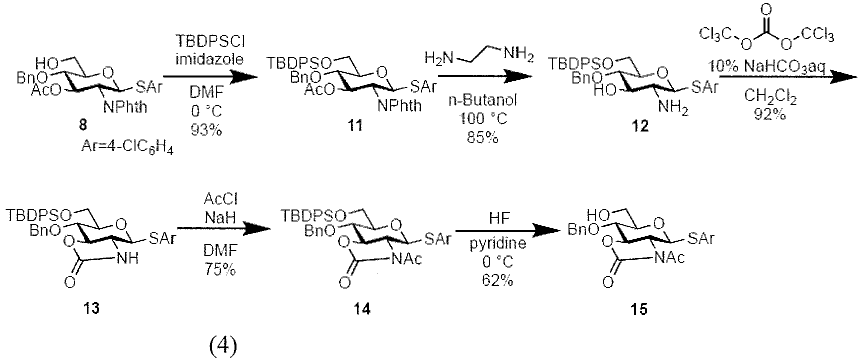

### [Synthesis of oxazolidinone protective component]

A polymerization reaction was performed by electrolytic oxidation using the oxazolidinone protective component 15 (reaction scheme 5). As a result, cyclic disaccharide 16 was selectively obtained with a yield of 60%. However, cyclic tri- or higher saccharides were not obtained. This is thought to be because, in cyclization and elongation of sugar chains, cyclization, which is an intramolecular reaction, is an advantageous reaction, and in electrolytic polymerization via the highly reactive primary hydroxyl group at the 6-position, synthesis of macrocyclic oligosaccharide is not expected.

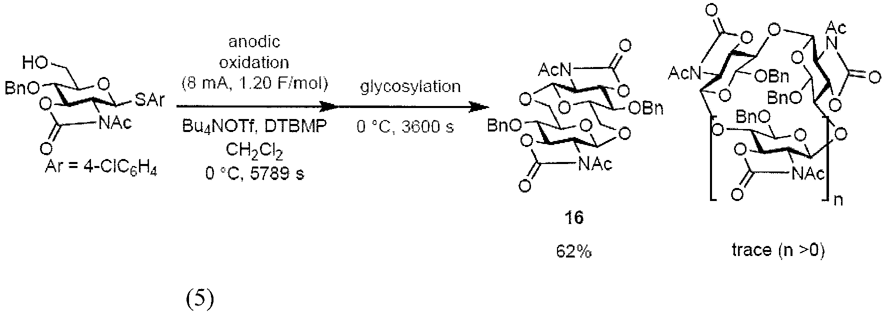

### [Electrolytic polymerization reaction using oxazolidinone protective component]

### 1,4-Bond cyclic saccharide polymerization reaction using oxazolidinone substrate

Since it was found that large cyclic oligosaccharides could not be obtained in electrolytic glycosylation polymerization via 1,6-glycosidic bonds, the approach was changed (reaction scheme 6). With this approach, large cyclic oligosaccharides could be obtained by glycosylation via 1,4-glycosidic bonds. An overview thereof is as follows.

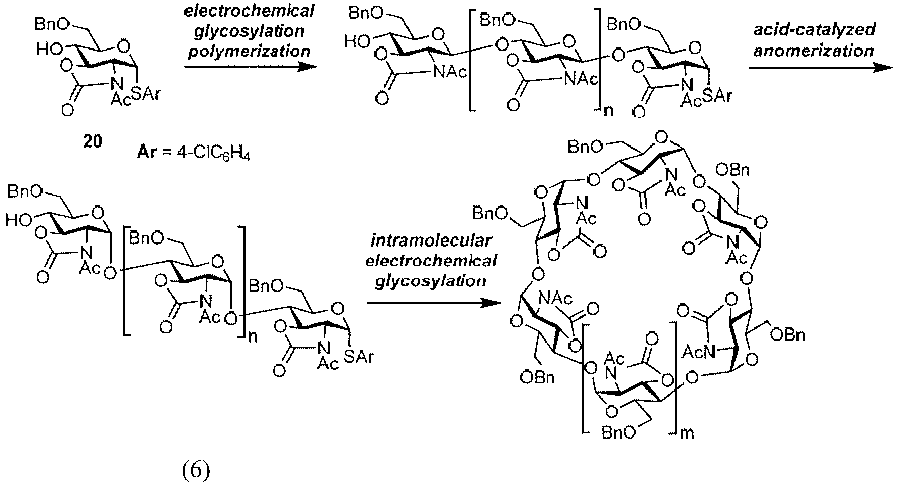

### [Overview of newly studied synthesis of large cyclic oligosaccharides (refer to the following literature)]

·Nokami, T.; Shibuya, A.; Saigusa, Y.; Manabe, S.; Ito, Y.; Yoshida, J. Beilstein J. Org. Chem. 2012, 8, 456.
·Benakli, K.; Zha, C.; Kerns, R. J. J. Am. Chem. Soc. 2001, 123, 9461.

In this approach, sugar chains were first extended via β-1,4-glycosidic bonds according to electrolytic polymerization, and the sugar chains were then isomerized according to specific α isomerization of the oxazolidinone protecting group. Then, cyclization was performed by electrolytic oxidation again. Using this approach, cyclic oligosaccharides were successfully synthesized rapidly and easily. It is particularly advantageous in that it can be performed by one-pot synthesis.

First, synthesis of the substrate will be described (reaction scheme 7). Using the substrate 6 synthesized above as a starting material, dephthaloylation was performed to obtain 17, and 2,3-oxazolidinone protection was then performed to obtain 18. This 18 was reacted with acetic anhydride in CH₂Cl₂ to obtain 19. This 19 was selectively subjected to ring-opening at the 4-position according to a benzylidene ring-opening reaction to synthesize a substrate 20.

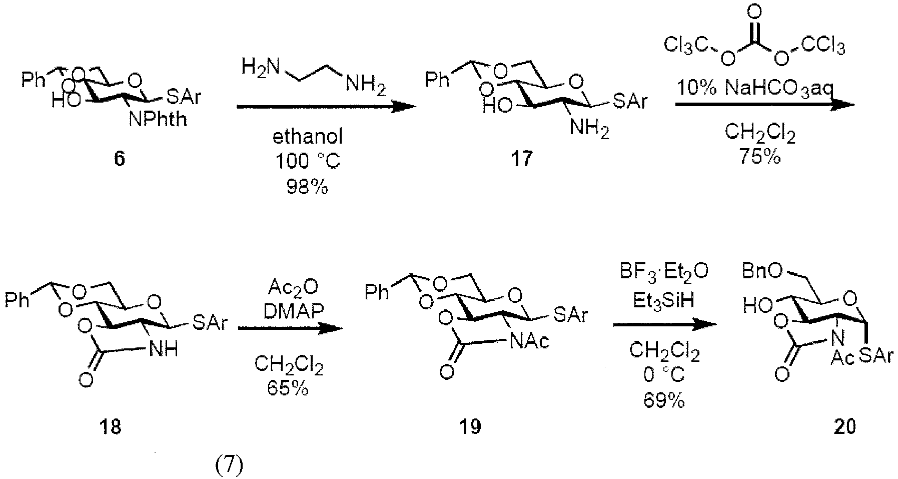

### [Synthesis of 2,3-oxazolidinone protective component with unprotected hydroxyl group at 4-position (refer to the following literature)]

·Feng, J.; Hevey, R.; Ling, C. Carbohydr. Res 2011, 346, 2650.

Based on this substrate 20, the sugar chain elongation step was optimized (Table 1 below). In this study, the temperature during electrolytic oxidation, and the presence of a base were mainly studied. The supporting electrolyte was fixed with Bu₄NOTf, the solvent was CH₂Cl₂, and the following conditions were studied. DTBMP, which is a bulky weak base, was used as the base.

### [Study of optimization of sugar chain elongation reaction conditions]

**[Table 1]**

| 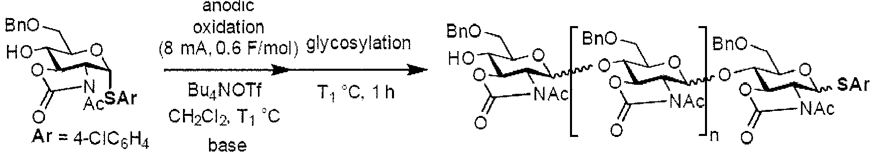 | | | | | | | | | |
|---|---|---|---|---|---|---|---|---|---|
| Entry | Base | T₁ (°C) | Yields of oligosaccharides (%) | | | | | | |
| | | | n = 0 | n = 1 | n = 2 | n = 3 | n = 4 | **Total** | Conv. |
| 1 | DTBMP | -60 | 21 | 9 | 3 | 5 | 2 | **40** | 75 |
| 2 | DTBMP | -40 | 14 | 8 | 9 | 6 | 1 | **38** | 77 |
| 3 | none | -40 | 22 | 18 | 9 | 5 | 4 | **58** | 78 |

### Base

### Yield of oligosaccharide

As a result, Entry 3 with no base was used as the subsequent conditions. The reason is that base-free conditions and high temperatures were more advantageous for sugar chain elongation. One-pot polymerization was performed by combining these conditions (reaction scheme 8). First, electrolytic oxidation and glycosylation were performed at -40C°, and the temperature was then raised to room temperature. In this case, sugar chains were isomerized for 1 hour using trifluoromethanesulfonic acid generated in the system by electrolytic oxidation. Then, the temperature was lowered to -40C° again, and electrolytic oxidation and glycosylation were performed. According to this experiment, the isolation yield of cyclic oligosaccharide with hexasaccharides 21 (cyclic hexasaccharide 21) was 4.8%, and the isolation yield of cyclic oligosaccharide with heptasaccharides 22 (cyclic heptasaccharide 22) was 0.6%. In addition, all the glycosidic bonds of the obtained cyclic oligosaccharides were α bonds.

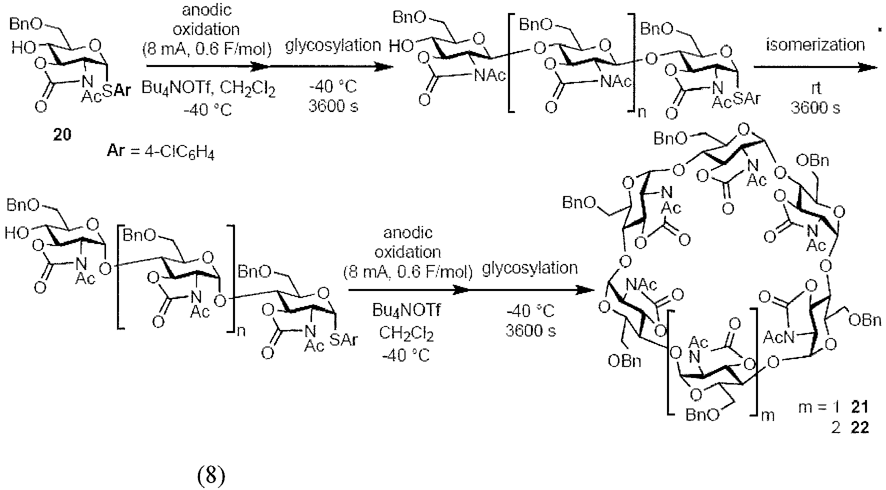

### isomerization

### [Reaction conditions for synthesis of one-pot cyclic oligosaccharide]

Next, changes in the states of these sugar chains are shown (chemical formula and NMR spectrum 24). First, it can be understood that the peak of α anomer hydrogen of a raw monosaccharide appeared at around 6.1 ppm, which was measured by 1H NMR.

When electrolytic polymerization was performed to elongate sugar chains, the peak of α anomer hydrogen was split into two types at around 6.0 ppm to 6.2 ppm, and at the same time, the peak of α anomer hydrogen could also be confirmed around 5.0 ppm. Then, when the chain oligosaccharide was treated with acid isomerization, the peak of α anomer hydrogen around 5.0 ppm disappeared. Finally, upon cyclization, the β anomer peak converged and shifted to around 5.8 ppm. It is known that, when an oligosaccharide is cyclized, the peak of anomer hydrogen shifts in a high magnetic field (Wakao, M.; Fukase, K.; and Kusumoto, S. J. Org. Chem. 2002. 67, 8182), and based on this, it is determined that it is a cyclic oligosaccharide.

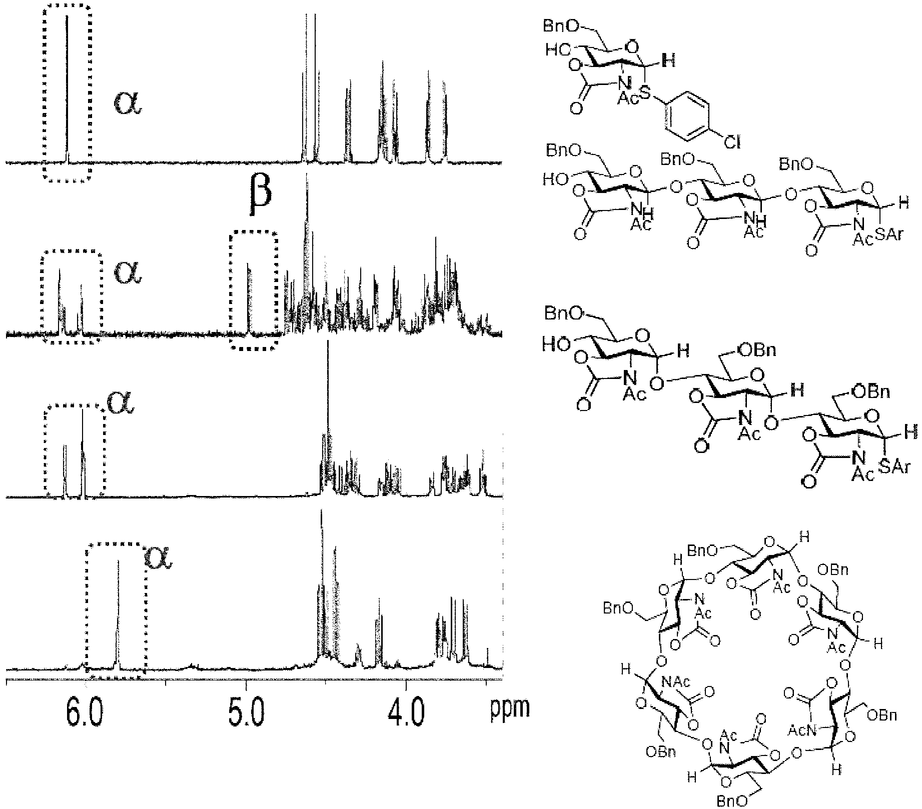

### [Measurement of sugar chain transition by chemical shift of anomer hydrogen in ¹H NMR]

The following invention was completed through the above development study results and examples to be described below.

That is, the invention provides a method for producing a cyclic oligosaccharide including subjecting a linear oligosaccharide in which 5 to 10 α-glucosamines or their derivatives are bonded in series via 1,4-bonds to a liquid phase electrolysis reaction.

In this case, the linear oligosaccharide is preferably represented by the following Formula (1). 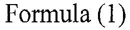 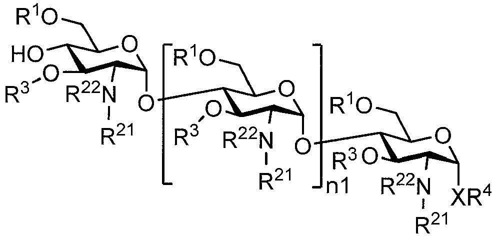 (in Formula (1), R¹'s are each independently a protecting group with a formula weight of 500 or less, R²¹ and R²² are each independently a hydrogen atom or a protecting group with a formula weight of 300 or less, R³'s are each independently a protecting group with a formula weight of 300 or less, R⁴ is a substituent, X is a sulfur atom, a selenium atom, or a tellurium atom, and n1 is an integer of 3 to 8; and R²² and R³ that are bonded to the same ring may be bonded to each other to form a ring)

In Formula (1), R¹'s are each independently a protecting group with a formula weight of 500 or less, and serve as a protecting group that protects a hydroxyl group. When a protecting group is provided, the progress of reactions other than cyclization can be curbed, and the cyclization reaction can progress effectively. In addition, when a protecting group with a formula weight of 500 or less is used, cyclization can be facilitated.

The formula weight of R¹ is preferably 60 or more, more preferably 80 or more, still more preferably 90 or more, yet more preferably 100 or more, and most preferably 105 or more. On the other hand, the upper limit is 500 or less, preferably 400 or less, more preferably 350 or less, still more preferably 300 or less, and yet more preferably 280 or less. Examples of preferable protecting groups include a Bn group, a benzoyl group (Bz), an acetyl group (Ac), a pivalic group (Piv), TBDPS, a tert-butyldimethylsilyl group (TBS), and a 9-fluorenylmethylcarboxy group (Fmoc), and Bn, TBDPS, and TBS are preferable, Bn and TBDPS are more preferable, and Bn is still more preferable.

R¹ may contain only one type or may contain two or more types.

R³ is a protecting group, and any type can be used as long as it does not inhibit cyclization of the α-1,4 chain sugar. In addition, when a protecting group with a formula weight of 300 or less is used, cyclization can be facilitated. On the other hand, when the protecting group weighs too little, it may not serve as a protecting group.

In view of this, the formula weight of R³ is preferably 20 or more, more preferably 30 or more, still more preferably 40 or more, and yet more preferably 50 or more. On the other hand, the upper limit is 300 or less, preferably 250 or less, more preferably 200 or less, still more preferably 180 or less, and yet more preferably 160 or less. When R³ has a too large formula weight, the cyclization reaction is inhibited. On the other hand, when the substituent weighs too little, it may not be able to sufficiently perform its role. Specific examples thereof include Bn, Bz, Ac, Piv, TBDPS, TBS, and Fmoc, and Bz, Ac, Piv, and Fmoc are preferable, Bz and Ac are more preferable, and Ac is still more preferable.

R³ may contain only one type or may contain two or more types.

R²¹ and R²² are a hydrogen atom or a protecting group, and any type can be used as long as it does not inhibit cyclization of the α-1,4 chain sugar. R²¹ and R²² are preferably a protecting group. In the case of a protecting group, cyclization can be facilitated using a protecting group with a formula weight of 300 or less. On the other hand, when the protecting group weighs too little, it may not serve as a protecting group. In view of this, in the case of a protecting group, the formula weight of R²¹ and R²² is preferably 20 or more, more preferably 30 or more, still more preferably 40 or more, and yet more preferably 50 or more. On the other hand, the upper limit is 300 or less, preferably 250 or less, more preferably 200 or less, still more preferably 180 or less, and yet more preferably 160 or less. Examples of preferable protecting groups include Bn, Bz, Ac, Piv, TBDPS, TBS, Fmoc, and a phthalic anhydride group (Phth), and Bn, Bz, Ac, Piv, TBDPS, TBS, Fmoc, and Phth are preferable, Bn, Ac, and Phth are more preferable, and Bn and Ac are still more preferable.

R²¹ and R²¹ each may contain only one type or may contain two or more types.

When R²¹ or R²², and R³ are bonded to form a ring, this is preferable because the glycosidic bond is easily isomerized into an α-1,4 bond and there is no effect on cyclization. The ring to be formed may be of any type as long as it does not inhibit cyclization of the α-1,4 bond chain sugar. As a specific ring to be formed, it is preferable to form a 5-membered or 6-membered heterocycle, and it is more preferable to form a 5-membered heterocycle. Specifically, it is preferable to form a morpholine ring, a morpholinone ring, or an oxazolidinone ring, and particularly preferable to form an oxazolidinone ring.

R⁴ is a substituent, and is preferably one that is electrochemically activated or can be activated by X such as sulfur. Particularly, R⁴ is preferably one that does not prevent oxidation of sulfur atoms and the like. R⁴ is preferably not an electron-withdrawing group but is an electron-donating group. However, in order to synthesize a chain oligosaccharide, an electron-withdrawing group is somewhat better. In view of this, the formula weight of R⁴ is preferably 20 or more, more preferably 30 or more, still more preferably 40 or more, and yet more preferably 50 or more. On the other hand, the upper limit is preferably 300 or less, more preferably 250 or less, still more preferably 200 or less, yet more preferably 180 or less, and most preferably 160 or less. Examples of preferable protecting groups include a substituent, a group with weaker electron-withdrawing properties than a nitro group, and an electron-donating group, specific examples thereof include a phenyl group, a 4-methylphenyl group, a 2,6-dimethylphenyl group, a 4-bromophenyl group, a 4-chlorophenyl group, and a 4-fluorophenyl group, a phenyl group, a 2,6-dimethylphenyl group, a 4-chlorophenyl group, and a 4-fluorophenyl group are preferable, a 2,6-dimethylphenyl group, a 4-chlorophenyl group, and a 4-fluorophenyl group are more preferable, and a 4-chlorophenyl group and a 4-fluorophenyl group are still more preferable.

n1 is an integer of 3 to 8, and preferably an integer of 4 to 6.

The cyclic or chain oligosaccharide may have a repeating structure of a single sugar or a repeating structure of different sugars as long as the definition in the formula is satisfied. This also applied to the following Formula (1-1), Formula (2), Formula (2-1), Formula (3), and Formula (4).

The linear oligosaccharide is preferably one represented by the following Formula (1-1). 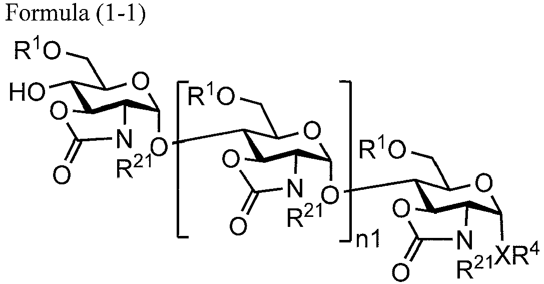 (in Formula (1-1), R¹'s are each independently a protecting group with a formula weight of 500 or less, R²¹'s are each independently a hydrogen atom or a protecting group with a formula weight of 300 or less, R⁴ is a substituent, X is a sulfur atom, a selenium atom, or a tellurium atom, and n1 is an integer of 3 to 8)

The preferable ranges of R¹, R²¹, R⁴, X and n1 are the same as those shown in Formula (1).

The liquid phase electrolysis reaction can be performed by other known methods as long as a linear oligosaccharide in which 5 to 10 α-glucosamines or their derivatives are bonded in series via 1,4-bonds is used.

For example, as the electrolytic solution, CH₂Cl₂, acetonitrile, propionitrile, DMF or the like can be used, and CH₂Cl₂ is preferable. As the supporting electrolyte, Bu₄NOTf, Et₄NOTf, Pr₄NOTf or the like can be used and Bu₄NOTf is preferable. The supporting electrolyte concentration is preferably in a range of 0.1 to 3.0 M and more preferably in a range of 0.5 to 2.0 M. The electrolytic oxidation temperature is preferably in a range of -100 to 0°C and more preferably in a range of -60 to -10°C.

The cyclic oligosaccharide obtained by the method for producing a cyclic oligosaccharide contains a novel compound. First, a cyclic oligosaccharide represented by Formula (2) can be exemplified. 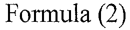 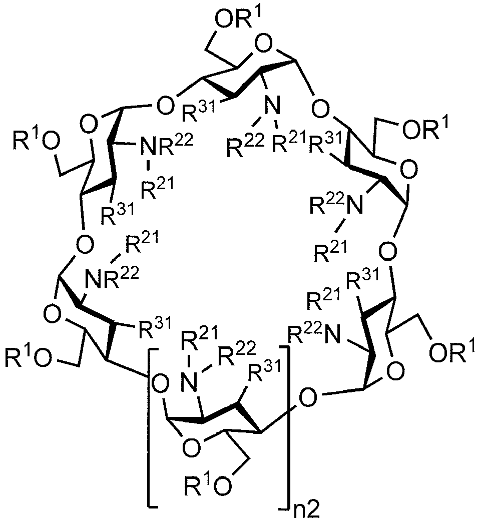 (in Formula (2), R¹'s are each independently a protecting group with a formula weight of 500 or less, R²¹ and R²² are each independently a hydrogen atom or a protecting group with a formula weight of 300 or less, R³¹ is an R³-O- group, R³'s are each independently a protecting group with a formula weight of 300 or less, and n2 is an integer of 0 to 5; and R²² and R³¹ that are bonded to the same ring may be bonded to each other to form a ring)

In Formula (2), the preferable ranges of R¹, R²¹, and R³ are the same as those shown in Formula (1). The ring formed by R²² and R³¹ also has the same meaning, and it is particularly preferable to form an oxazolidinone ring.

In addition, as the novel compound, a cyclic oligosaccharide represented by Formula (2-1) may be exemplified. 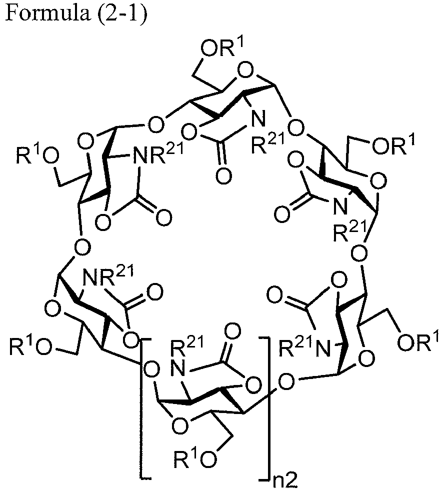 (in Formula (2-1), R¹'s are each independently a protecting group with a formula weight of 500 or less, R²¹'s are each independently a hydrogen atom or a protecting group with a formula weight of 300 or less, and n2 is an integer of 0 to 5)

R¹ and R²¹ in Formula (2-1) are the same as those defined in Formula (1).

n2 is preferably an integer of 0 to 3, more preferably 1 or 2, and still more preferably 1.

In addition, as the novel compound, a cyclic oligosaccharide represented by Formula

### (3) may be exemplified.

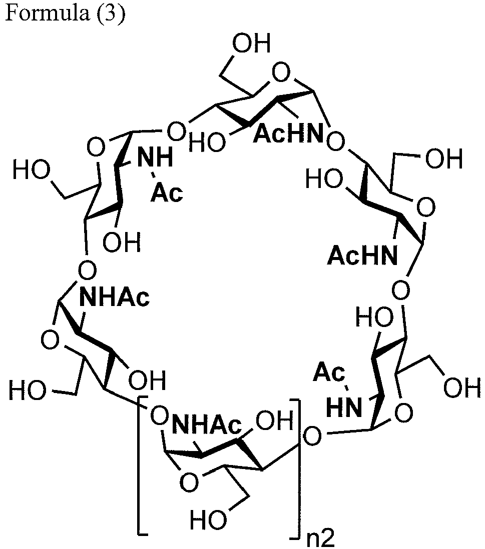

(in Formula (3), n2 represents an integer of 0 to 5, and Ac represents an acetyl group)

n2 is preferably an integer of 0 to 3, more preferably 1 or 2, and still more preferably 1.

The compound represented by Formula (3) can be derived from the compound represented by Formula (2-1). Specifically, first, the oxazolidinone ring is opened with a base (for example, sodium hydroxide). The obtained sugar is reacted with acetic anhydride and DMAP (4-dimethylaminopyridine). In addition, by reacting with K₂CO₃, a sugar in which an acetyloxy group is converted into a hydroxyl group is obtained. This can be reacted with an acid (preferably, reacted with an acid under a hydrogen atmosphere) to convert Bn (benzyl group) into a hydrogen atom, and a sugar of Formula (3) in which the substituent at the 6-position is a hydroxyl group can be obtained.

In addition, a cyclic oligosaccharide represented by the following Formula (4) may be exemplified. 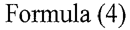 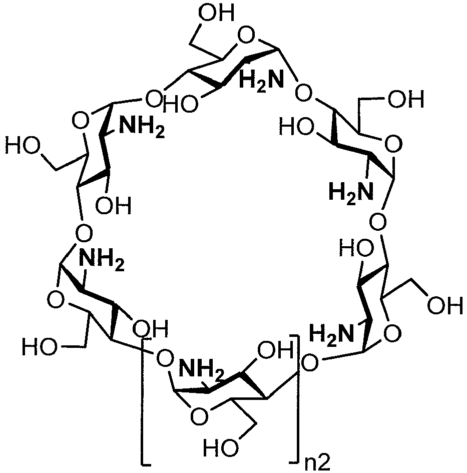 (in Formula (4), n2 represents an integer of 0 to 5)

n2 is preferably an integer of 0 to 3, more preferably 1 or 2, and still more preferably 1.

The compound represented by Formula (4) can be obtained by deacetylation of the compound represented by Formula (3). Deacetylation can be performed by treatment with a base. Examples of bases here include lithium hydroxide, sodium hydroxide, potassium hydroxide, sodium methoxide, and hydroxylamine.

The novel compounds represented by Formulae (2) to (4) are useful as inclusion compounds. For example, they can be used for the same purposes as cyclodextrin, and are useful as an inclusion agent or an encapsulating agent. Particularly, cyclodextrins with 6- to 8-membered rings are widely synthesized and industrialized. Since the novel compounds represented by Formulae (2) to (4) contain an amino group, they are excellent in inclusion of those having an acidic substituent such as a carboxylic acid group.

### [Examples]

### Liquid phase electrolysis automatic synthesis device

In a preferable embodiment of the present invention, oligosaccharides were synthesized by a liquid phase electrolysis automatic synthesis method. In this method, activation was performed by electrolytically oxidizing a glycosyl donor (reaction scheme 9). Unlike conventional glycosylation methods such as Schmidt glycosylation, this method is advantageous in that the reaction can be controlled more precisely because intermediates can be accumulated. Using this precision and reproducibility, based on an electrolytic polymerization method in which a substrate had a role of both a glycosyl donor and a glycosyl acceptor, an efficient cyclic oligosaccharide synthesis method was developed.

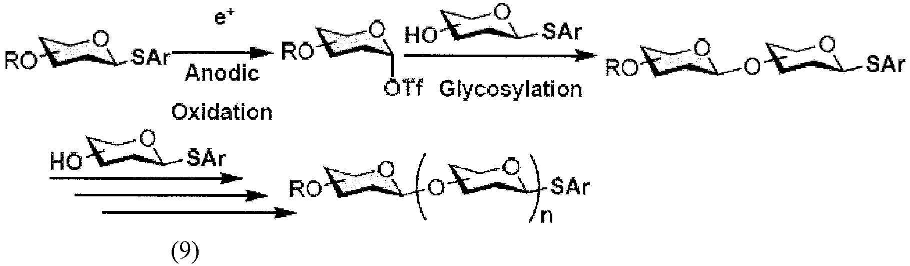

### [Synthesis of oligosaccharide by liquid phase electrolysis automatic synthesis device (refer to the following literature)]

Nokami, T.; Shibuya, A.; Tsuyama, H.; Suga. S.; Albert A. Bowers, Crich,D.; Yoshida, J. J. Am. Chem. Soc. 2007, 129, 10922.

In 2019, a second generation liquid phase automatic electrolysis device was developed. This device has an advantage that reaction conditions can be controlled more easily and a desired compound can be synthesized in a larger amount. In the present invention, this second generation liquid phase automatic electrolysis device was used to develop an efficient cyclic oligosaccharide synthesis method.

Hereinafter, the present invention will be described in more detail with reference to examples. Materials, usage amounts, ratios, treatment details, treatment procedures and the like shown in the following examples can be appropriately changed without departing from the spirit of the present invention. Therefore, the scope of the present invention is not limited to specific examples shown below.

When measurement instruments and the like used in examples are difficult to obtain due to discontinuation or the like, measurement can be performed using other instruments having the same performance.

Regarding the reagent, all commercially available reagents were used. Regarding the solvent, all dehydrated solvents were used. During the glycosylation reaction, 4 Å molecular sieves were added to the dehydrated solvent under an argon atmosphere, and additional dehydration was performed.

Here, in the following examples, acetylated compounds are exemplified, but acetylated compounds can be easily deacetylated by treatment with a base.

Nuclear magnetic resonance spectrums were measured using Bruker AVANCE II 600 (1H NMR; 600 MHz, 13C NMR; 150 MHz) and CDCl₃ as a solvent at room temperature.

### <Preparation Example 101>

### Synthesis of 1,3,4,6-penta-O-acetyl-2-deoxy-2-phthalimido-β-D-glucopyranoside (3)

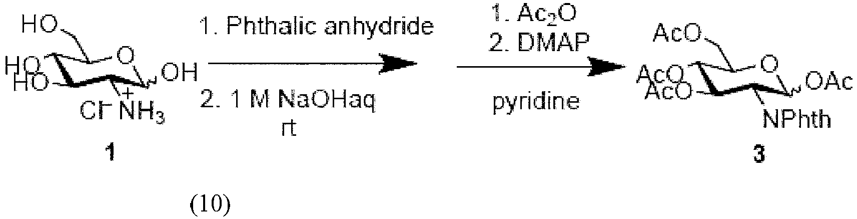

A compound 1 (20.0 g, 92.8 mmol) was put into a 500 mL flask and dissolved in 120 mL of a 1 M sodium hydroxide aqueous solution. Then, phthalic anhydride (16.32 g, 110.7 mmol) was added and the mixture was stirred overnight. Then, completion of the reaction was confirmed using TLC (MeOH), and the mixture was concentrated and vacuum-dried. Then, a mixture containing DMAP (1.22 g, 9.9 mmol) as a catalyst was dissolved in pyridine (300 mL) under an argon atmosphere, acetic anhydride (597.6 mmol, 57.0 mL) was added, and the mixture was reacted for 2 days.

Then, completion of the reaction was confirmed using TLC (Hexane:EtOAc=1/1), and the reaction was terminated with MeOH. In order to remove the solvent, the crude product dissolved in EtOAc (ethyl acetate) was separated and washed three times with 1 M HClaq, NaHCOsaq, and H₂O in that order, dried with Na₂SO₄, filtered, concentrated, and vacuum-dried. The obtained crude product was produced with a silica gel column (Hexane/EtOAc=1/1) to obtain a compound 3.
yield amount 22.0 g, 46.2 mmol yield 50%

### <Preparation Example 102>

### Synthesis of 4-Chlorophenyl 3,4,6-tri-O-acetyl-2-deoxy-2-phthalimido-1-thio-β-D-glucopyranoside (4)

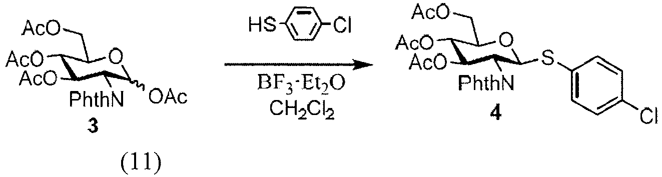

A compound 3 (22.04 g, 46.7 mmol) was put into a flask, 4-Chlorothiophenol (8.04 g, 55.4 mmol) was added thereto, and the flask was purged with argon. CH₂Cl₂ (68.4 mL) was added and the mixture was stirred. BF₃/OEt₂ (8.55 mL, 69.3 mmol) was added dropwise at 0°C. The mixture was stirred overnight at 50°C. TLC check (Hexane/EtOAc=1:1) was performed. NaHCO₃aq was added for quenching. The organic layer was extracted with CH₂Cl₂ three times. The organic layer was washed with desalted water three times and dehydrated with Na₂SO₄. Filtration was performed and concentration was performed. The sample obtained by dissolution in EtOAc, recrystallization with Hexane, and filtration was vacuum-dried to obtain a compound 4.
yield amount 21.2 g, 37.7 mmol yield 81%

### <Preparation Example 103>

### Synthesis of 4-Chlorophenyl 4,6-O-benzylidene-2-deoxy-2-phthalimido-1-thio-β-D-glucopyranoside (6)

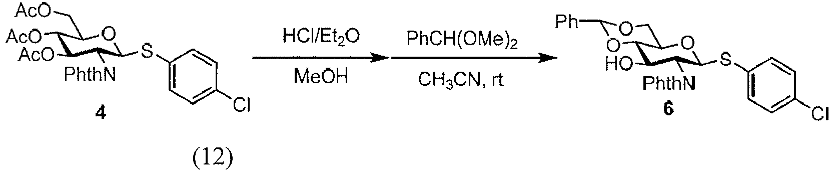

A compound 4 (10.00 g, 17.8 mmol) was put into an eggplant flask, the flask was purged with argon, and MeOH (87.0 mL) was added thereto. 2.0 M HCl/Et₂O was added and the mixture was stirred for 3 days. Concentration was performed and vacuum-drying was performed. The flask was purged with argon, and CH₃CN (96.2 mL) was added thereto. Benzaldehyde Dimethyl Acetal (65.2 mmol, 9.76 mL) was added dropwise and the mixture was stirred for 2 days. TLC check (Hexane/EtOAc=2:1) was performed. Et₃N (4.2 mL) was added for quenching. Concentration was performed and fractioning with a silica gel column (Hexane/EtOAc=2:1) was performed. Concentration was performed, and vacuum-drying was performed.
yield amount 6.18 g, 11.8 mmol yield 66% (2 steps)

### <Preparation Example 104>

### Synthesis of 4-Chlorophenyl-3-O-acetyl-4,6-O-benzylidene-2-deoxy-2-phthalimido-1-thio-β-Dglucopyranoside (7)

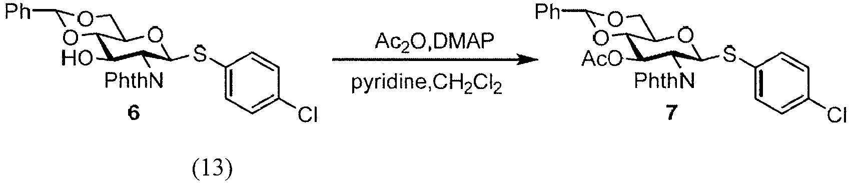

DMAP (0.14 g, 1.22 mmol) was added to an eggplant flask containing a compound 6 (5.84 g, 11.4 mmol), and the flask was purged with argon. CH₂Cl₂ (37.2 mL) and pyridine (11.8 mL) were added. Ac₂O (72.2 mmol, 6.69 mL) was added dropwise, and the mixture was stirred overnight at room temperature. TLC check (Hexane/EtOAc=2:1) was performed. Methanol was added to terminate the reaction. Concentration was performed, and vacuum-drying was performed. Dissolution in CH₂Cl₂ was performed, and Hexane was added for recrystallization. Filtration was performed and vacuum-drying was performed to obtain a compound 7.
yield amount 4.78 g, 8.45 mmol yield 76%

### <Preparation Example 105>

### Synthesis of 4-Chlorophenyl 3-O-acetyl-4-O-benzyl-2-deoxy-2-phtalimido-1-thio-β-Dglucopyranoside (8)

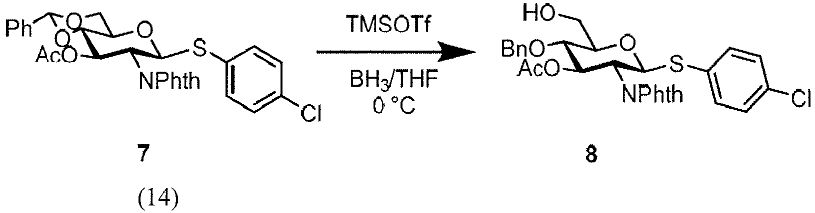

A compound 7 (4.78 g, 8.45 mmol) was put into an eggplant flask and vacuum-dried, the flask was then purged with argon, BH₃/THF (21.2 mL) was added, and TMSOTf (2.12 mL) was added dropwise at 0°C. The mixture was stirred under a condition of 0°C for 6 hours. After completion of the reaction was confirmed using TLC (Hexane/EtOAc=2/1), Et₃N (10 mL) and Methanol (24 mL) were added dropwise in that order to terminate the reaction, and concentration and vacuum-drying were performed. The crude product was dissolved in EtOAc, and subjected to 3 sets of separation and washing with NaHCO₃aq and desalted water, and dried with Na₂SO₄. Filtration, concentration, and vacuum-drying were performed, and purification with a silica gel column (Hexane/EtOAc=2/1 to 2/1) was performed to obtain a compound 8.
yield amount 2.80 g, 49.4 mmol yield 58%

### <Reference Example 101>

### Electrolytic polymerization using compound 8

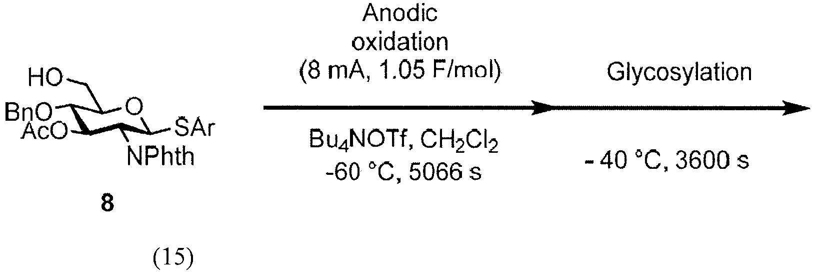

The inside of a 10 mL H-type separation electrolysis cell was purged with argon, Bu4NOTf (1.0 mmol, 0.392 g) was added to the anode, and 8 (0.40 mmol, 0.248 g) was added thereto. Bu₄NOTf (1.0 mmol, 0.392 g) was put into the cathode. The inside of the cell was evacuated, and vacuum-dried overnight. The inside of the cell was purged with argon, and 10 mL of CH₂Cl₂ was added to each of the cathode and the anode, and TfOH (40 µL) was added to the cathode. Electrolytic oxidation was performed for 5,066 s at 8.0 mA and -60°C. Glycosylation was performed for 3,600 s at -40°C. 0.3 mL of Et₃N was added to each of the cathode and the anode for quenching. Concentration and vacuum-drying were performed and washing with H₂O was performed. The obtained crude product was dissolved in 3 mL of CH₂Cl₂ and purified by GPC.

### <Preparation Example 201>

### Synthesis of 4-Chlorophenyl 3-O-acetyl-4-O-benzyl-6-O-tert-butyldimethyldiphenylsilyl-2-deoxy-2-phtalimido1-thio-β-D-glucopyranoside (11)

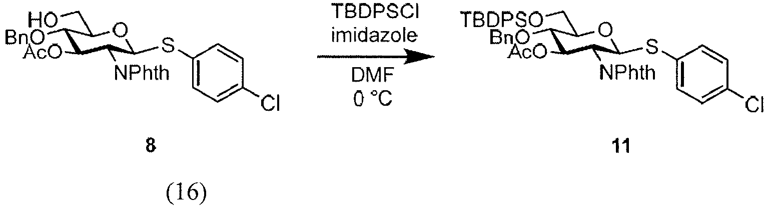

The compound 8 (3.42 g, 6.02 mmol) and imidazole (0.89 g, 12.0 mmol) were put into an eggplant flask and vacuum-dried, and the flask was then purged with argon, and dissolution in DMF (21.8 mL) was performed at 0°C. Then, TBDPSCl (2.64 mL) was added dropwise and the mixture was stirred overnight at room temperature. After completion of the reaction was confirmed using TLC (Hexane:EtOAc=3/1), dilution with EtOAc was performed, separation and washing with NaHCO₃aq and H₂O were performed three times, and drying with Na₂SO₄ was performed. Then, filtration, concentration, and vacuum-drying were performed, and purification with a silica gel column (Hexane:EtOAc=3/1) was performed to obtain a compound 11.
yield amount 4.52 g, 5.60 mmol yield 93%

### <Preparation Example 202>

### Synthesis of 4-Chlorophenyl 2-amino-6-O-tert-butyldimethyldiphenylsilyl-2-deoxy-1-thio-β-D-glucopyranoside (12)

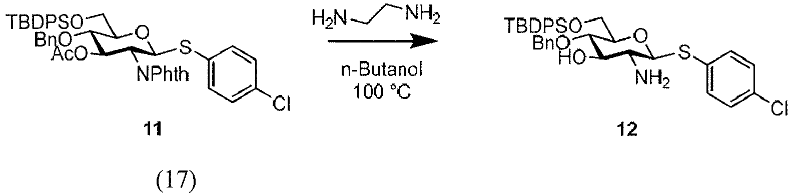

A compound 11 (4.52 g, 5.60 mmol) was put into an eggplant flask and vacuum-dried, the flask was then purged with argon, and dissolution in n-butanol (37.5 mL) was performed. Then, ethylene diamine anhydrous (9.37 mL) was added dropwise, and the temperature was raised to reflux in the order of 30°C, 50°C, 80°C, and 100°C. Then, the mixture was stirred overnight and completion of the reaction was confirmed using TLC (Hexane/EtOAc=1/2). Then, the solvent was concentrated and vacuum-dried, and the crude product was purified with a silica gel column (Hexane/EtOAc=1/2) to obtain a compound 12.
yield amount 3.07 g, 4.77 mmol yield 85%

### <Preparation Example 203>

### Synthesis of 4-Chlorophenyl 6-O-tert-butyldimethyldiphenylsilyl-2-deoxy-2,3-N,O-carbonyl-1-thio-β-D-glucopyranoside (13)

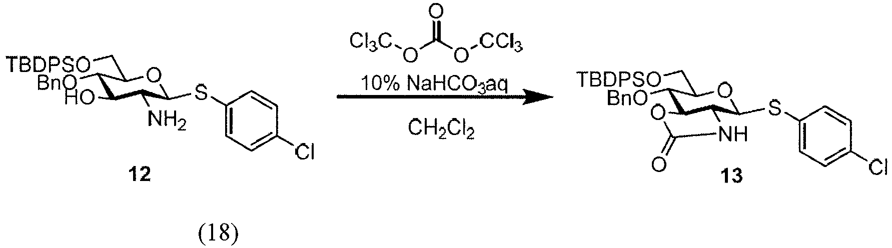

The compound 12 (3.02 g, 4.77 mmol) and Triphosgene (0.56 g, 1.90 mmol) were put into an eggplant flask, and dissolved in CH₂Cl₂ (133.3 mL) under an air atmosphere. Then, 10% NaHCO₃aq (99.7 mL) was added, and the mixture was stirred overnight. Then, dilution and extraction with CH₂Cl₂ were performed. Then, the extract solution was washed with H₂O, and dried with Na₂SO₄. Then, filtration, concentration, and vacuum-drying were performed to obtain a compound 13.
yield amount 2.89 g, 4.37 mmol yield 92%

### <Preparation Example 204>

### Synthesis of 4-Chlorophenyl 2-acetamido-6-O-tert-butyldimethyldiphenylsilyl-2-deoxy-2,3-N,Ocarbonyl-1-thio-β-D-glucopyranoside (14)

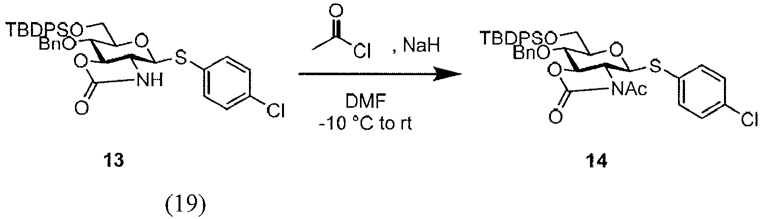

A compound 13 (2.89 g, 4.37 mmol) and 60% NaH (1.22 g, 51.2 mmol) were put into an eggplant flask and vacuum-dried, and the flask was purged with argon. Then, dissolution in DMF (24.3 mL) was performed, acetylchloride (2.4 mL) was added dropwise at 0°C, and the mixture was stirred overnight. Then, completion of the reaction was confirmed using TLC (Hexane/EtOAc=3/1), and quenching with NaHCO₃aq was performed. Then, dilution with CH₂Cl₂ was performed, separation and washing with NaHCO₃aq and H₂O were performed three times, and drying with Na₂SO₄ was performed. Then, concentration and vacuum-drying were performed, and purification with a silica gel column (Hexane/EtOAc=3/1) was performed to obtain a compound 14.

### <Preparation Example 205>

### Synthesis of 4-Chlorophenyl 2-acetamido-2-deoxy-2,3-N,O-carbonyl-1-thio-β-D-glucopyranoside (15)

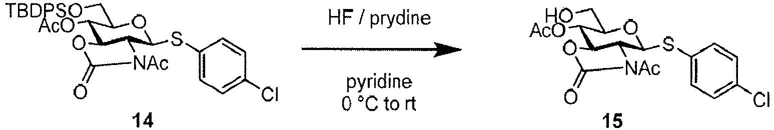

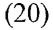

A compound 14 (2.30 g, 3.28 mmol) was put into a plastic flask and vacuum-dried, the flask was then purged with argon, pyridine was added, and 30% HF/pyridine was added dropwise at 0°C. Then, the mixture was stirred for 4 hours, and NaHCO₃aq was added for quenching. Then, dissolution in EtOAc was performed, separation and washing with H₂O were performed, and drying with Na₂SO₄ was performed. Filtration, concentration, and vacuum-drying were performed, and purification with a silica gel column (Hexane/EtOAc=3/1) was performed to obtain a compound 15.
yield amount 0.94 g, 2.03 mmol yield 62%

### <Reference Example 201>

### Electrolytic polymerization using compound 15

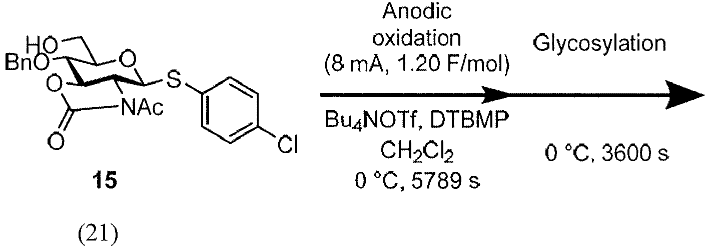

The inside of a 10 mL H-type separation electrolysis cell was purged with argon, and Bu₄NOTf (1.0 mmol, 0.392 g) and DTBMP (2.0 mmol, 0.410 g) were added to the anode, and 8 (0.40 mmol, 0.185 g) was added thereto. Bu₄NOTf (1.0 mmol, 0.392 g) was put into the cathode. The inside of the cell was evacuated, and vacuum-dried overnight. The inside of the cell was purged with argon, and 10 mL of CH₂Cl₂ was added to each of the cathode and the anode, and TfOH (40 µL) was added to the cathode. Electrolytic oxidation was performed for 5,790 s at 8.0 mA and 0°C. Glycosylation was performed for 3,600 s at 0°C. 0.3 mL of Et₃N was added to each of the cathode and the anode for quenching. Concentration and vacuum-drying were performed, and washing was performed three times with HClaq, NaHCO₃aq, and H₂O. The obtained crude product was dissolved in 3 mL of CH₂Cl₂ and purified by GPC. As a result, it was confirmed that cyclic oligosaccharide with disaccharide 16 was produced (refer to NMR spectrums below and Figs. 1 to 4).

### Cyclic Disaccharide 16

[α]²⁴D = 43.5 (c = 0.7, CHCl₃). ¹H-NMR (600 MHz, CDCl₃): δ 7.38-7.35 (m, 4 H), 7.33-7.30 (m, 1 H), 5.25-5.23 (d, J = 5.7 Hz, 1 H), 4.91-4.89 (d, J = 11.4 Hz, 1 H), 4.63-4.61(d, J = 11.2 Hz, 1 H), 4.33-4.29 (dd, J = 12.5, 9.8 Hz, 1 H), 4.19-4.17 (dd, J = 9.6, 4.3 Hz,1 H), 4.15-4.14 (dd, J = 2.4, 1.0 Hz, 1 H), 4.14 (m, 1 H), 4.02-4.00 (dd, J = 10.9, 2.5 Hz,1 H), 3.95-3.92 (dd, J = 12.5, 5.8 Hz, 1 H), 3.60-3.58 (d, J = 10.5 Hz, 1 H), 2.53 (s, 3 H).¹³C-NMR (150 MHz, CDCl₃): δ 170.6, 153.7, 137.3, 128.5, 128.0, 127.9, 97.0, 81.7, 73.1,63.8, 62.0, 24.5; HRMS (ESI) m/z calcd for C₃₂H₃₄KN₂O₁₂ [M+K]⁺, 677.1744; found,677.1735.

### <Preparation Example 301>

### Synthesis of 4-Chlorophenyl 2-amino-4,6-O-benzylidene-2-deoxy-1-thio-β-D-glucopyranoside (17)

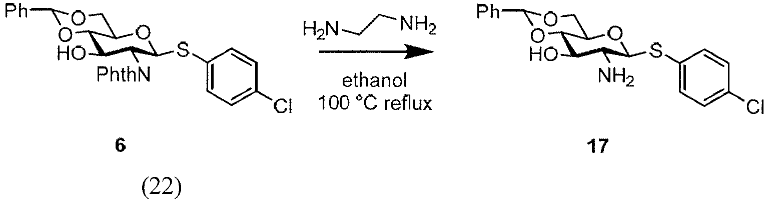

A compound 6 (8.96 g, 17.1 mmol) was put into an eggplant flask vacuum-dried, and the flask was then purged with argon, and dissolution in ethanol (90 mL) was performed. Then, ethylene diamine anhydrous (18.6 mL) was added dropwise, and the temperature was raised to reflux in the order of 30°C, 50°C, 80°C, and 100°C. Then, the mixture was stirred overnight, and completion of the reaction was confirmed using TLC (CH₂Cl₂/MeOH=8/1). Then, the solvent was concentrated and vacuum-dried, and the crude product was purified with a silica gel column (CH₂Cl₂/MeOH=8/1) to obtain 17.
yield amount 6.56 g, 16.7 mmol yield 98%

### <Preparation Example 302>

### Synthesis of 4-Chlorophenyl 4,6-O-benzylidene-2-deoxy-2,3-N,O-carbonyl-1-thio-β-D-glucopyranoside (18)

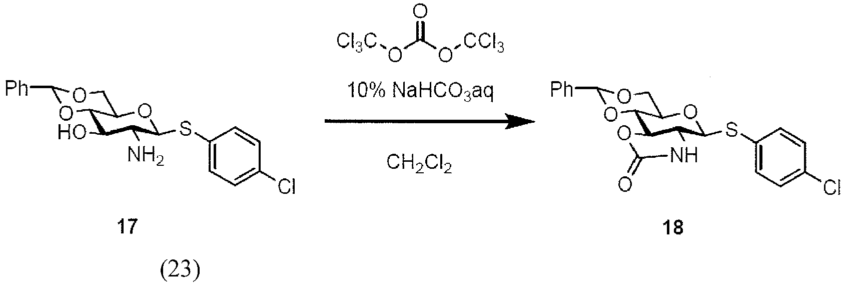

17 (6.56 g, 16.7 mmol) and Triphosgene (1.69 g, 5.68 mmol) were put into an eggplant flask and dissolved in CH₂Cl₂ (250 mL) under an air atmosphere. Then, 10% NaHCO₃aq (187 mL) was added and the mixture was stirred overnight. Then, dilution and extraction with CH₂Cl₂ were performed. Then, the extract solution was washed with H₂O, and dried with Na₂SO₄. Then, filtration, concentration, and vacuum-drying were performed to obtain 18.
yield amount 5.34 g, 12.7 mmol yield 76%

### <Preparation Example 303>

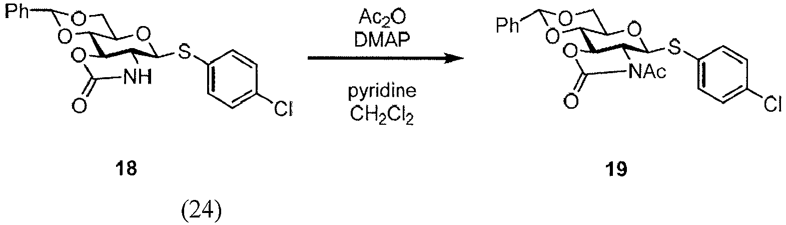

A compound 18 (5.34 g, 12.7 mmol) and DMAP (0.31 g, 2.54 mmol) were put into an eggplant flask and vacuum-dried, the flask was then purged with argon, and CH₂Cl₂ (38 mL) and pyridine (12.3 mL) were added. Then, acetic anhydride (11.8 mL) was added dropwise, and the mixture was stirred overnight. Then, completion of the reaction was confirmed using TLC (Hexane/EtOAc=2:1) and quenching with MeOH was performed. Then, concentration and vacuum-drying were performed. A small amount of MeOH was added to the obtained crude product, and the mixture was left for 1 day, washed and purified to obtain a compound 19.
yield amount 3.80 g, 8.22 mmol yield 65%

### <Preparation Example 304>

### Synthesis of 4-Chlorophenyl 2-acetamido-6-O-benzyl-2-deoxy-2,3-N,O-carbonyl-1-thio-β-D-glucopyranoside (20)

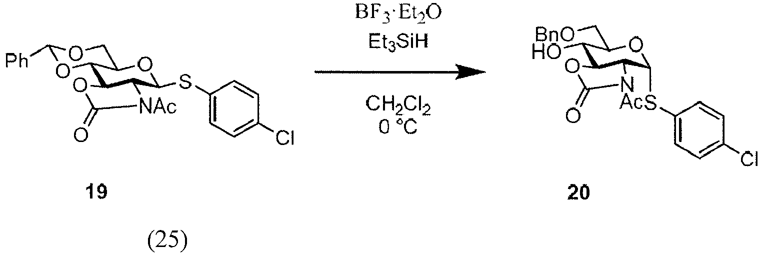

A compound 19 (3.51 g, 7.61 mmol) was put into an eggplant flask and vacuum-dried, the flask was then purged with argon, and CH₂Cl₂ (119.9 mL) and triethyl silane (14.6 mL) were added. Then, BF₃/Et₂O (1.4 mL) was added dropwise, and the mixture was stirred for 4 hours. Then, NaHCO₃aq was added for quenching, separation and washing with H₂O were performed, and drying with Na₂SO₄ was performed. Then, filtration, concentration, and vacuum-drying were performed, and purification with a silica gel column (Hexane/EtOAc=2/1) was performed to obtain a compound 20.
yield amount 2.44 g, 5.25 mmol yield 69%

### <Example 301>

### Electrolytic polymerization using compound 20

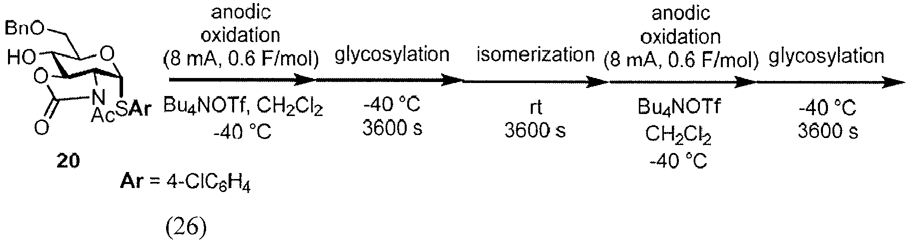

The inside of a 10 mL H-type separation electrolysis cell was purged with argon, Bu4NOTf (1.0 mmol, 0.392 g) was added to the anode, and 8 (0.40 mmol, 0.185 g) was added thereto. Bu₄NOTf (1.0 mmol, 0.392 g) was put into the cathode. The inside of the cell was evacuated, and vacuum-dried overnight. The inside of the cell was purged with argon, and 10 mL of CH₂Cl₂ was added to each of the cathode and the anode, and TfOH (40 µL) was added to the cathode. Electrolytic oxidation was performed for 2,895 s at 8.0 mA and -40°C. Glycosylation was performed for 3,600 s at -40°C. Then, the temperature was raised to room temperature, and the mixture was stirred for 3,600 s. Then, again, electrolytic oxidation was performed for 2,895 s at 8.0 mA and -40°C. Glycosylation was performed for 3,600 s at -40°C. 0.5 mL of Et₃N was added to each of the cathode and the anode for quenching. Concentration and vacuum-drying were performed, and washing with H₂O was performed. The obtained crude product was dissolved in 3 mL of CH₂Cl₂ and purified by GPC. The obtained fraction containing a cyclic hexasaccharide was dissolved in CH₃Cl, fractionated by preparative TLC (Hexane: EtOAc=2:3), and a portion around Rf=0.7 was scraped off and eluted with CH₃Cl. As a result, it was confirmed that a cyclic oligosaccharide with hexasaccharides 21 was produced (refer to NMR spectrums below and Figs. 5 to 8).

### Cyclic hexasaccharide 21

¹H-NMR (600 MHz, CDCl₃): δ 7.33-7.26 (m, 5 H), 5.80-5.80 (d, J = 2.4 Hz, 1 H), 4.54-4.49 (m, 2 H), 4.44-4.42 (d, J = 11.8 Hz, 1 H), 4.18-4.15 (pseudo-t, J = 9.0 Hz, 1 H), 3.81-3.79 (dd, J = 10.7, 4.5 Hz, 1 H), 3.77-3.75 (dd, J = 9.2, 4.9 Hz, 1 H), 3.71-3.69 (dd, J =12.2, 2.3 Hz, 1 H), 3.64-3.62 (d, 10.0 Hz, 1 H), 2.53 (s, 3 H). ¹³C-NMR (150 MHz, CDCl₃): δ 172.1, 152.3, 137.4, 128.5, 128.0, 127.8, 98.0, 77.7, 74.8, 74.4, 73.7, 68.0, 59.6, 23.5;HRMS (ESI) m/z calcd for C₉₆H₁₀₂N₆NaO₃₆ [M+Na]⁺, 1938.6261; found, 1938.6140.

### <Example 302>

### Synthesis of cyclic hexasaccharide 26 from cyclic hexasaccharide 21

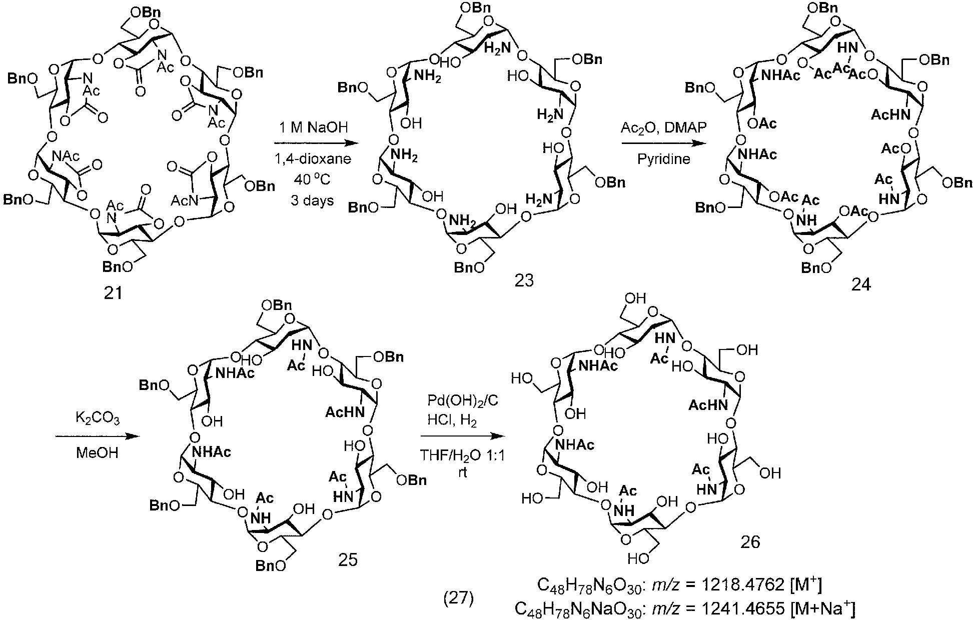

### Synthesis of Cyclohexakis-(1,4)-(2-amino-6-O-benzyl-2-deoxy-α-D-glucopyranosyl) (23)

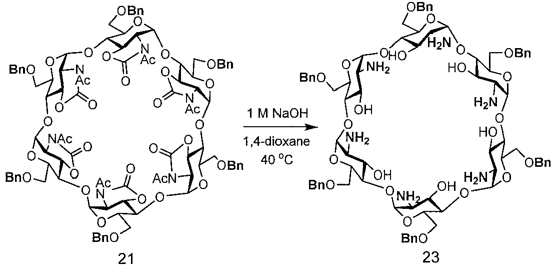

A compound 21 (37.7 mg, 0.0197 mmol) was put into a glass eggplant flask and vacuum-dried, the flask was then purged with argon, and 1.4 mL of 1,4-dioxane was added. 1.4 mL of a NaOH aqueous solution whose concentration was adjusted to 1 M was added dropwise thereto and the mixture was stirred for 1 day. Then, the progress of the reaction was confirmed using MALDI-TOF-MS, and concentration, vacuum-drying, and freeze-drying were then performed to obtain 85.7 mg of a solid containing a desired saccharide 23.

### Synthesis of Cyclohexakis-(1,4)-(2-acetamide-3-O-acetyl-6-O-benzyl-2-deoxy-α-D-glucopyranosyl) (24)

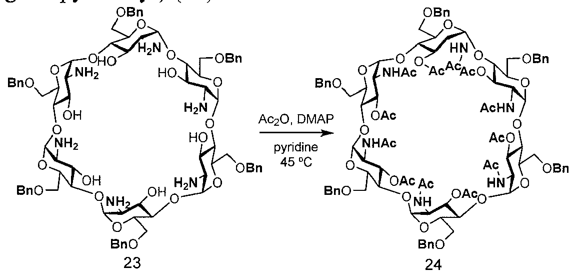

A solid containing the saccharide 23 (85.7 mg) and DMAP (5.61 mg, 3.04 mmol) were put into a glass eggplant flask and vacuum-dried, and the flask was then purged with argon. 3.0 mL of pyridine was added thereto, 0.28 mL of acetic anhydride was added dropwise, and the mixture was stirred at 45°C for 7 days. Then, MeOH was added for quenching. Then, concentration and vacuum-drying were performed. The obtained solid was dissolved in EtOAc and separated and washed with H₂O. The organic layer was dried with Na₂SO₄, concentrated, and vacuum-dried to obtain 47.2 mg of a crude product. This crude product was purified by column chromatography (Hexane/EtOAc (volume ratio)=2:1, and then CH₂Cl₂/MeOH (volume ratio)=1:1) to obtain a saccharide 24 (22.4 mg, 0.0111 mmol) with a yield of 56% (2 steps).

### Synthesis of Cyclohexakis-(1,4)-(2-acetamide-6-O-benzyl-2-deoxy-α-D-glucopyranosyl) (25)

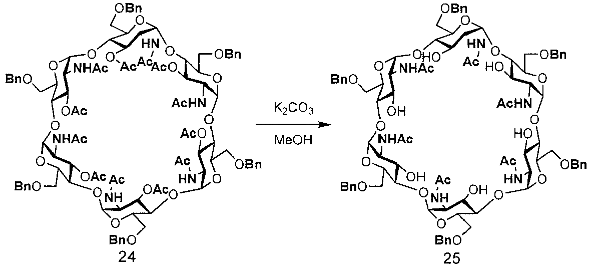

A saccharide 24 (22.4 mg, 0.0111 mmol) and K₂CO₃ (14.8 mg, 0.111 mmol) were put into a glass eggplant flask and vacuum-dried, and the flask was then purged with argon. Here, 2.32 mL of a mixed solvent containing CH₂Cl₂/MeOH (1:2) was added and the mixture was stirred for 1 day. The progress of the reaction was confirmed using MALDI-TOF-MS, and concentration and vacuum-dry were then performed to obtain a solid containing a desired product 25 (46.2 mg).

### Synthesis of Cyclohexakis-(1,4)-(2-acetamide-2-deoxy-α-D-glucopyranosyl) (26)

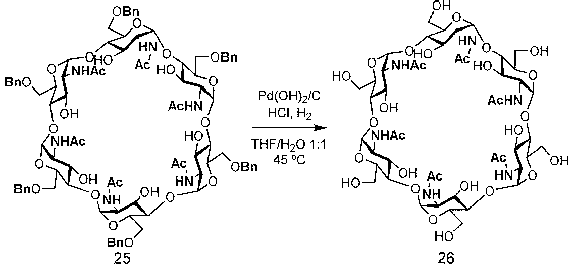

The solid containing a saccharide 25 in the glass eggplant flask was vacuum-dried and purging with argon was then performed. Here, 1.00 mL of a mixed solvent containing THF/H₂O (1:1) was added. Then, freezing was performed once, 70 mg of Pd (OH)₂/C was added, vacuum-drying was performed, and purging with hydrogen was then performed. The mixture was stirred for 7 days, the progress of the reaction was confirmed using MALDI-TOF-MS, and concentration and vacuum-drying were then performed to obtain a solid containing a desired product. This solid was purified by Sphadex LH-20, gel filtration column chromatography to obtain a saccharide 26 (1.6 mg, 0.0013 mmol) with a yield of 12% (2 steps).

Fig. 9 shows MALDI-TOF MS after reaction termination in a reaction scheme (27). Fig. 10 shows MALDI-TOF MS after gel filtration (Sephadex LH-20, solvent: deionized water) of a product. Fig. 11 shows ¹H-NMR after gel filtration of a product. Based on these results, it was found that a desired product (compound of Formula (3)) was obtained.

Fig. 12 shows mass spectrometry data of the saccharide 24, which is an intermediate. The calculated value and measured value are as follows.
mass spectrometry (MALDI-TOF) C102H126N6NaO36 [M+Na+] calculated value: 2033.811, measured value: 2033.807

Fig. 13 shows mass spectrometry data of the saccharide 26, which is the final product. The calculated value and measured value are as follows.
mass spectrometry (MALDI-TOF) C48H78N6NaO30 [M+Na+] calculated value: 1241.466, measured value: 1241.466

## Claims

1. A method for producing a cyclic oligosaccharide, the method comprising subjecting a linear oligosaccharide, in which 5 to 10 α-glucosamines or derivatives thereof are bonded in series via 1,4-bonds, to a liquid phase electrolysis reaction.

2. The method for producing a cyclic oligosaccharide according to claim 1,
wherein the linear oligosaccharide is represented by Formula (1) below: 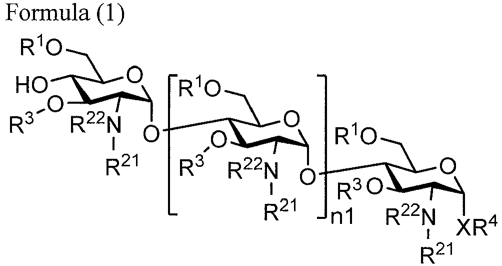 (in Formula (1), R¹'s are each independently a protecting group with a formula weight of 500 or less, R²¹ and R²² are each independently a hydrogen atom or a protecting group with a formula weight of 300 or less, R³'s are each independently a protecting group with a formula weight of 300 or less, R⁴ is a substituent, X is a sulfur atom, a selenium atom, or a tellurium atom, and n1 is an integer of 3 to 8; and R²² and R³ that are bonded to the same ring may be bonded to each other to form a ring).

3. The method for producing a cyclic oligosaccharide according to claim 1,
wherein the linear oligosaccharide is represented by Formula (1-1) below: 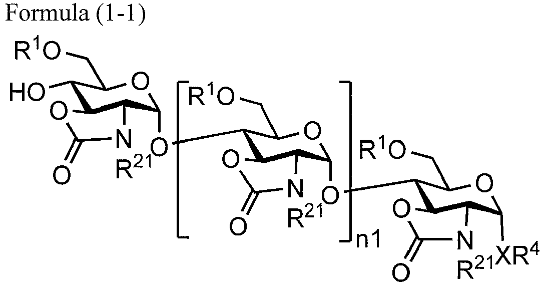 (in Formula (1-1), R¹'s are each independently a protecting group with a formula weight of 500 or less, R²¹'s are each independently a hydrogen atom or a protecting group with a formula weight of 300 or less, R⁴ is a substituent, X is a sulfur atom, a selenium atom, or a tellurium atom, and n1 is an integer of 3 to 8).

4. A cyclic oligosaccharide represented by Formula (2): 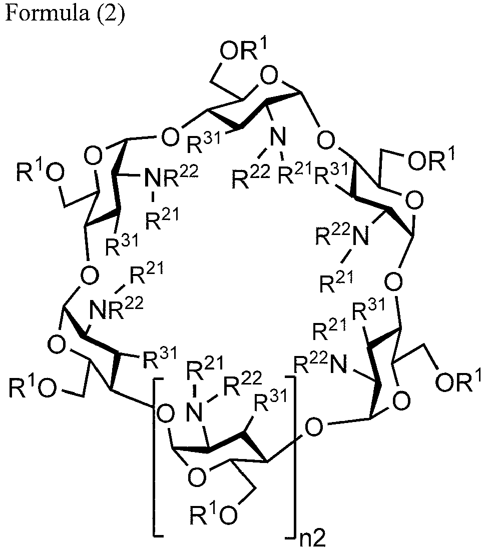 (in Formula (2), R¹'s are each independently a protecting group with a formula weight of 500 or less, R²¹ and R²² are each independently a hydrogen atom or a protecting group with a formula weight of 300 or less, R³¹ is an R³-O- group, protecting groups R³'s are each independently a protecting group with a formula weight of 300 or less, and n2 is an integer of 0 to 5; and R²² and R³¹ that are bonded to the same ring may be bonded to each other to form a ring).

5. A cyclic oligosaccharide represented by Formula (2-1): 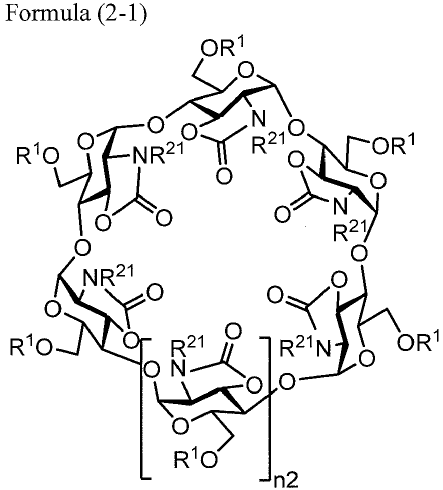 (in Formula (2-1), R¹'s are each independently a protecting group with a formula weight of 500 or less, R²¹'s are each independently a hydrogen atom or a protecting group with a formula weight of 300 or less, and n2 is an integer of 0 to 5).

6. A cyclic oligosaccharide represented by Formula (3): 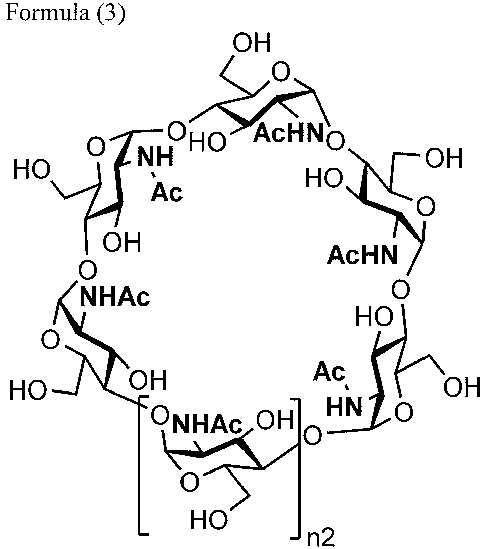 (in Formula (3), n2 represents an integer of 0 to 5, and Ac represents an acetyl group).

7. A cyclic oligosaccharide represented by Formula (4): 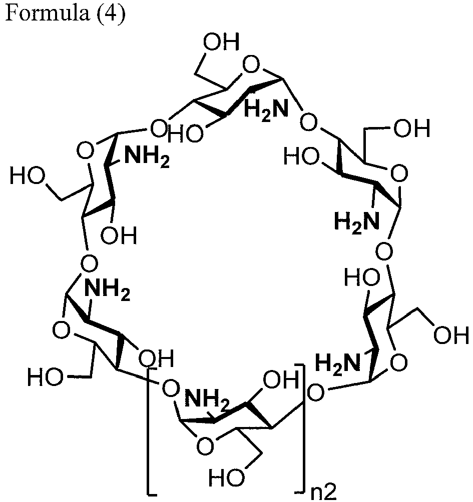 (in Formula (4), n2 represents an integer of 0 to 5).

8. An inclusion agent containing the compound according to claim 7.

9. A method for producing a cyclic oligosaccharide, the method comprising
opening an oxazolidinone ring of a cyclic oligosaccharide represented by Formula (2-1) below with a base, reacting an obtained sugar with acetic anhydride, acetylating the ring-opened site, additionally reacting with K₂CO₃ to obtain a sugar in which an acetyloxy group at the 3-position is converted into a hydroxyl group, reacting the sugar with an acid to obtain a sugar of Formula (3) in which a group for R¹ is a hydrogen atom and the substituent at the 6-position is a hydroxyl group: 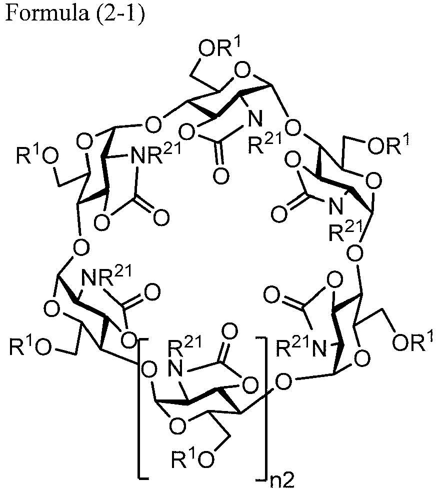 (in Formula (2-1), R¹'s are each independently a protecting group with a formula weight of 500 or less, R²¹'s are each independently a hydrogen atom or a protecting group with a formula weight of 300 or less, and n2 is an integer of 0 to 5) 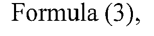 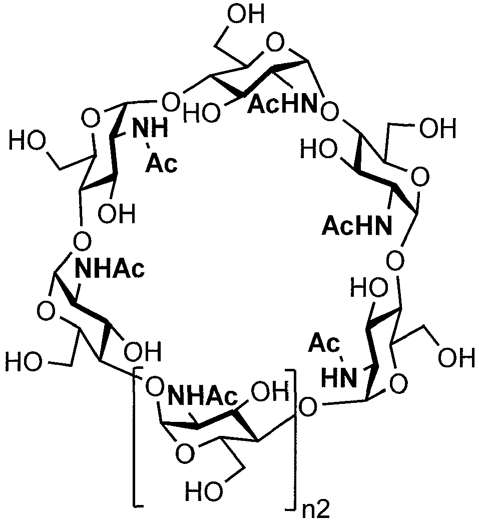 (in Formula (3), n2 represents an integer of 0 to 5, and Ac represents an acetyl group).
